(19) Europäisches Patentamt
European Patent Office
Office européen des brevets

(11)  EP 2 737 081 B1

(12)  EUROPEAN PATENT SPECIFICATION

(45) Date of publication and mention
of the grant of the patent:
**28.09.2016  Bulletin 2016/39**

(21) Application number: **12740974.6**

(22) Date of filing: **30.07.2012**

(51) Int Cl.:
**C12Q 1/68** (2006.01)

(86) International application number:
**PCT/EP2012/064865**

(87) International publication number:
**WO 2013/014296 (31.01.2013 Gazette 2013/05)**

(54) **METHOD FOR PREDICTING THE RESPONSE TO CHEMOTHERAPY IN A PATIENT SUFFERING FROM OR AT RISK OF DEVELOPING RECURRENT BREAST CANCER**

METHODE ZUR VORHERSAGE DES ANSPRECHENS AUF CHEMOTHERAPIE IN PATIENTEN MIT REZIDIVIERENDEM BRUSTKREBS ODER ENTSPRECHENDEM RISIKO

MÉTHODE DE PRÉDICTION DE LA RÉPONSE À UNE CHIMIOTHÉRAPIE D'UN PATIENT SOUFFRANT DE OU ÉTANT À RISQUE DE DÉVELOPPER UN CANCER DU SEIN RÉCURRENT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:  **28.07.2011  EP 11175852**

(43) Date of publication of application:
**04.06.2014  Bulletin 2014/23**

(60) Divisional application:
**16184484.0**

(73) Proprietor: **Sividon Diagnostics GmbH**
**50829 Köln (DE)**

(72) Inventors:
• **GEHRMANN, Mathias**
**51368 Leverkusen (DE)**
• **WEBER, Karsten**
**50829 Cologne (DE)**
• **KRONENWETT, Ralf**
**50829 Cologne (DE)**
• **PETRY, Christoph**
**50829 Cologne (DE)**
• **BRASE, Jan**
**50829 Cologne (DE)**

(74) Representative: **dompatent von Kreisler Selting Werner -**
**Partnerschaft von Patent- und Rechtsanwälten mbB**
**Deichmannhaus am Dom**
**Bahnhofsvorplatz 1**
**50667 Köln (DE)**

(56) References cited:
**WO-A1-2006/119593        WO-A1-2009/114836**
**WO-A1-2009/158143        WO-A1-2010/076322**
**WO-A2-2008/006517**

• **MAÏA CHANRION ET AL: "A gene expression signature that can predict the recurrence of tamoxifen-treated primary breast cancer", CLINICAL CANCER RESEARCH, THE AMERICAN ASSOCIATION FOR CANCER RESEARCH, US, vol. 14, no. 6, 15 March 2008 (2008-03-15) , pages 1744-1752, XP002634156, ISSN: 1078-0432, DOI: 10.1158/1078-0432.CCR-07-1833**
• **"Affymetrix Genechip bHuman Genome U133 plus 2.0 Array", 7 November 2003 (2003-11-07), GEO, XP002343693, the whole document**
• **TAYLOR KAREN J ET AL: "Dynamic changes in gene expression in vivo predict prognosis of tamoxifen-treated patients with breast cancer", BREAST CANCER RESEARCH, CURRENT SCIENCE, LONDON, GB, vol. 12, no. 3, 22 June 2010 (2010-06-22), page R39, XP021085352, ISSN: 1465-5411, DOI: 10.1186/BCR2593**

**Description**

**Technical Field**

The present invention relates to methods, kits and systems for predicting the response of a tumor to chemotherapy. More specific, the present invention relates to the prediction of the response to chemotherapeutic agents, in particular but not limited to a neoadjuvant setting based on the measurements of gene expression levels in tumor samples of breast cancer patients.

**Background of the Invention**

Breast cancer is the most common tumor type and one of the leading causes of cancer-related death in women (Jemal et al., CA Cancer J Clin., 2011). It is estimated that every tenth woman will develop breast cancer during her lifetime. Although the incidence has increased over the years, the mortality has constantly decreased due to the advances in early detection and the development of novel effective treatment strategies.

Breast cancer patients are frequently treated with radiotherapy, hormone therapy or cytotoxic chemotherapy after surgery (adjuvant treatment) to control for residual tumor cells and reduce the risk of recurrence. Chemotherapy includes the combined use of several cytotoxic agents, whereas anthracycline and taxane-based treatment strategies have been shown to be superior compared to other standard combination therapies (Misset et al.,J Clin Oncol., 1996, Henderson et al., J Clin Oncol., 2003).

Systemic chemotherapy is commonly applied to reduce the likelihood of recurrence in HER2/neu-positive and in tumors lacking expression of the estrogen receptor and HER2/neu receptor (triple negative, basal). The most challenging treatment decision concerns luminal (estrogen receptor positive and HER2/neu-negative) tumors for which classical clinical factors like grading, tumor size or lymph node involvement do not provide a clear answer to the question whether to use chemotherapy or not.

To reduce the number of patients suffering from serious side effects without a clear benefit of systemic therapy, there is a great need for novel molecular biomarkers to predict the sensitivity to chemotherapy and thus allow a more tailored treatment strategy.

Chemotherapy can also be applied in the neoadjuvant (preoperative) setting in which breast cancer patients receive systemic therapy before the remaining tumor cells are removed by surgery. Neoadjuvant chemotherapy of early breast cancer leads to high clinical response rates of 70-90%. However, in the majority of clinical responders, the pathological assessment of the tumor residue reveals the presence of residual tumor cell foci. A complete eradication of cancer cells in the breast and lymph nodes after neoadjuvant treatment is called pathological complete response (pCR) and observed in only 10-25% of all patients. The pCR is an appropriate surrogate marker for disease-free survival and a strong indicator of benefit from chemotherapy.

The preoperative treatment strategy provides the opportunity to directly assess the response of a particular tumor to the applied therapy: the reduction of the tumor mass in response to therapy can be directly monitored. For patients with a low probability of response, other therapeutic approaches should be considered. Biomarkers can be analyzed from pretherapeutic core biopsies to identify the most valuable predictive markers. A common approach is to isolate RNA from core biopsies for the gene expression analysis before neoadjuvant therapy. Afterwards the therapeutic success can be directly evaluated by the tumor reduction and correlated with the gene expression data.

Predictive multigene assays like the DLDA30 (Hess et al., J Clin Oncol., 2006) have been shown to provide information beyond clinical parameters like tumor grading and hormone receptor status in breast cancer patients treated with neoadjuvant therapy. However, the predictive multigene test DLDA30 was established without considering the estrogen receptor status. Therefore the test might reflect phenotypic differences between complete responder and nonresponder, responders being predominantly ER-negative and HER2/neu positive (Tabchy et al., Clin Can Res, 2010).

Additionally, established multigene tests for prognosis were analyzed in the neoadjuvant setting to assess whether the prognostic assays can also predict chemosensitivity. One example is the Genomic Grade Index (GGI), a multigene test to define histologic grade based on gene expression profiles (Sotiriou et al, JNCI, 2006). It was demonstrated by Liedtke and colleagues that a high GGI is associated with increased chemosensitivity in breast cancer patients treated with neoadjuvant therapy (Liedtke, J Clin Oncol, 2009).

Although gene signatures have been shown to predict the therapy response, large-scale validation studies including clinical follow-up data are missing and so far none of them is commonly used to guide treatment decisions in clinical routine as yet.

WO2010/076322 A1 discloses a method for predicting a response to and/or benefit from chemotherapy in a patient suffering from cancer comprising the steps of (i) classifying a tumor into at least two classes, (ii) determining in a tumor sample the expression of at least one marker gene indicative of a response to chemotherapy for a tumor in each respective class, (iii) depending on said gene expression, predicting said response and/or benefit ; wherein said at least one marker gene comprises a gene selected from the group consisting of TMSL8, ABCC1, EGFR, MVP, ACOX2, HER2/NEU, MYH11, TOB1, AKR1C1, ERBB4, NFKB1A, TOP2A, AKR1C3, ESR1, OLFM1, TOP2B, ALCAM, FRAP1, PGR, TP53, BCL2, GADD45A, PRKAB1, TUBA1A, C16orf45, HIF1A, PTPRC, TUBB, CA12, IGKC, RACGAP1, UBE2C, CD14, 1KBKB, S100A7, VEGFA, CD247, KRT5, SEPT8, YBX1, CD3D, MAPK3, SLC2A1, CDKN1A, MAPT, SLC7A8, CHPT1, MLPH, SPON1, CXCL13, MMP1, STAT1, CXCL9, MMP7, STC2, DCN, MUC1, STMN1 and combinations thereof.

Maia Chanrion et al. report in Clin Cancer Res 2008; 14(6) March 15, 2008, p. 1744-1752 about a gene expression signature that can predict the recurrence of tamoxifen-treated primary breast cancer. The disclosed study identifies a molecular signature specifying a subgroup of patients who do not gain benefits from tamoxifen treatment. These patients may therefore be eligible for alternative endocrine therapies and/or chemotherapy.

WO 2009/158143A1 discloses methods for classifying and for evaluating the prognosis of a subject having breast cancer are provided. The methods include prediction of breast cancer subtype using a supervised algorithm trained to stratify subjects on the basis of breast cancer intrinsic subtype. The prediction model is based on the gene expression profile of the intrinsic genes listed in Table 1. This prediction model can be used to accurately predict the intrinsic subtype of a subject diagnosed with or suspected of having breast cancer. Further provided are compositions and methods for predicting outcome or response to therapy of a subject diagnosed with or suspected of having breast cancer. These methods are useful for guiding or determining treatment options for a subject afflicted with breast cancer. Methods of the invention further include means for evaluating gene expression profiles, including microarrays and quantitative polymerase chain reaction assays, as well as kits comprising reagents for practicing the methods of the invention

WO 2006/119593 discloses methods and systems for prognosis determination in tumor samples, by measuring gene expression in a tumor sample and applying a gene-expression grade index (GGI) or a relapse score (RS) to yield a numerical risk score

Karen J Taylor et al. report in Breast Cancer Research 2010, 12:R39 about dynamic changes in gene expression *in vivo* to predict prognosis of tamoxifen-treated patients with breast cancer.

WO 2008/006517A2 discloses methods and kits for the prediction of a lively outcome of chemotherapy in a cancer patient. More specifically, the invention relates to the prediction of tumor response to chemotherapy based on measurements of expression levels of a small set of marker genes. The set of marker genes is useful for the identification of breast cancer subtypes responsive to taxane based chemotherapy, such as e.g. a taxane-anthracycline-cyclophosphamide-based (e.g. Taxotere (docetaxel)- Adriamycin (doxorubicin)-cyclophosphamide, i.e. (TAC)-based) chemotherapy.

WO 2009/114836 A1 discloses gene sets which are useful in assessing prognosis and/or predicting the response of cancer, e.g. colorectal cancer to chemotherapy, are disclosed. Also disclosed is a clinically validated cancer test, e.g. colorectal test, for assessment of prognosis and/or prediction of patient response to chemotherapy, using expression analysis. The use of archived paraffin embedded biopsy material for assay of all markers in the relevant gene sets is accomodated for, and therefore is compatible with the most widely available type of biopsy material.

WO 2011/120984A1 discloses methods, kits and systems for the prognosis of the disease outcome of breast cancer, said method comprising : (a) determining in a tumor sample from said patient the RNA expression levels of at least 2 of the following 9 genes: UBE2C, BIRC5, RACGAP1, DHCR7, STC2, AZGP1, RBBP8, IL6ST, and MGP (b) mathematically combining expression level values for the genes of the said set which values were determined in the tumor sample to yield a combined score, wherein said combined score is indicative of a prognosis of said patient; and kits and systems for performing said method.

## Definitions

Unless defined otherwise, technical and scientific terms used herein have the same meaning as commonly understood by one of ordinary skill in the art to which this invention belongs.

"Predicting the response to chemotherapy", within the meaning of the invention, shall be understood to be the act of determining a likely outcome of cytotoxic chemotherapy in a patient affected by cancer. The prediction of a response is preferably made with reference to probability values for reaching a desired or non-desired outcome of the chemotherapy. The predictive methods of the present invention can be used clinically to make treatment decisions by choosing the most appropriate treatment modalities for any particular patient.

The "response of a tumor to chemotherapy", within the meaning of the invention, relates to any response of the tumor to cytotoxic chemotherapy, preferably to a change in tumor mass and/or volume after initiation of neoadjuvant chemotherapy and/or prolongation of time to distant metastasis or time to death following neoadjuvant or adjuvant chemotherapy. Tumor response may be assessed in a neoadjuvant situation where the size of a tumor after systemic intervention can be compared to the initial size and dimensions as measured by CT, PET, mammogram, ultrasound or palpation, usually recorded as "clinical response" of a patient. Response may also be assessed by caliper measurement or pathological examination of the tumor after biopsy or surgical resection. Response may be recorded in a quantitative fashion like percentage change in tumor volume or in a qualitative fashion like "no change" (NC), "partial remission" (PR), "complete remission" (CR) or other qualitative criteria. Assessment of tumor response may be done early after the onset of neoadjuvant therapy e.g. af-ter a few hours, days, weeks or preferably after a few months. A typical endpoint for response assessment is upon termination of neoadjuvant chemotherapy or upon surgical removal of residual tumor cells and/or the tumor bed. This is typically three month after initiation of neoadjuvanttherapy. Response may also be assessed by comparing time to distant metastasis or death of a patient following neoadjuvant or adjuvant chemotherapy with time to distant metastasis or death of a patient not treated with chemotherapy.

The term "tumor" as used herein, refers to all neoplastic cell growth and proliferation, whether malignant or benign, and all pre-cancerous and cancerous cells and tissues.

The term "cancer" refer to or describe the physiological condition in mammals that is typically characterized by unregulated cell growth. The term "cancer" as used herein includes carcinomas, (e.g., carcinoma in situ, invasive carcinoma, metastatic carcinoma) and pre-malignant con-ditions, neomorphic changes independent of their histological origin. The term "cancer" is not limited to any stage, grade, histomorphological feature, invasiveness, aggressiveness or malignancy of an affected tissue or cell aggregation. In particular stage 0 cancer, stage I cancer, stage II cancer, stage III cancer, stage IV cancer, grade I cancer, grade II cancer, grade III cancer, malignant cancer and primary carcinomas are included.

The term "cytotoxic chemotherapy" refers to various treatment modalities affecting cell proliferation and/or survival. The treatment may include administration of alkylating agents, antimetabolites, anthracyclines, plant alkaloids, topoisomerase inhibitors, and other antitumor agents, including monoclonal antibodies and kinase inhibitors. In particular, the cytotoxic treatment may relate to a taxane treatment. Taxanes are plant alkaloids which block cell division by preventing microtubule function. The prototype taxane is the natural product paclitaxel, originally known as Taxol and first derived from the bark of the Pacific Yew tree. Docetaxel is a semi-synthetic analogue, of paclitaxel. Taxanes enhance stability of microtubules, preventing the separation of chromosomes during anaphase.

The term "therapy" refers to a timely sequential or simultaneous administration of anti-tumor, and/or anti vascular, and/or anti strota, and/or immune stimulating or suppressive, and/or blood cell proliferative agents, and/or radiation therapy, and/or hyperthermia, and/or hypothermia for cancer therapy. The administration of these can be performed in an adjuvant and/or neoadjuvant mode. The composition of such "protocol" may very in the dose of each of the single agents, timeframe of application and frequency of administration within a defined therapy window. Currently various combinations of various drugs and/or physical methods, and various schedules are under investigation. A. "taxane/anthracycline-containing chemotherapy" is a therapy modality comprising the administration of taxane and/or anthracycline and therapeutically effective derivates thereof.

The term "neoadjuvant chemotherapy" relates to a preoperative therapy regimen consisting of a panel of hormonal, chemotherapeutic and/or antibody agents, which is aimed to shrink the primary tumor, thereby rendering local therapy (surgery or radiotherapy) less destructive or more effective, enabling breast conserving surgery and evaluation of responsiveness of tumor sensitivity towards specific agents in vivo.

The term "lymph node involvement" means a patient having previously been diagnosed with lymph node metastasis. It shall encompass both draining lymph node, near lymph node, and distant lymph node metastasis. This previous diagnosis itself shall not form part of the inventive method. Rather it is a precondition for selecting patients whose samples may be used for one embodiment of the present invention. This previous diagnosis may have been arrived at by any suitable method known in the art, including, but not limited to lymph node removal and pathological analysis, biopsy analysis, in-vitro analysis of biomarkers indicative for metastasis, imaging methods (e.g. computed tomography, X-ray, magnetic resonance imaging, ultrasound), and intraoperative findings.

The term "pathological complete response" (pCR), as used herein, relates to a complete disappearance or absence of invasive tumor cells in the breast and/or lymph nodes as assessed by a histopathological examination of the surgical specimen following neoadjuvant chemotherapy.

The term "marker" or "biomarker" refers to a biological molecule, e.g., a nucleic acid, peptide, protein, hormone, etc., whose presence or concentration can be detected and correlated with a known condition, such as a disease state.

The term "predictive marker" relates to a marker which can be used to predict the clinical response of a patient towards a given treatment.

The term "prognosis", as used herein, relates to an individual assessment of the malignancy of a tumor, or to the expected response if there is no drug therapy. In contrast thereto, the term "prediction" relates to an individual assessment of the malignancy of a tumor, or to the expected response if the therapy contains a drug in comparison to the malignancy or response without this drug.

The term "immunohistochemistry" or IHC refers to the process of localizing proteins in cells of a tissue section exploiting the principle of antibodies binding specifically to antigens in biological tissues. Immunohistochemical staining is widely used in the diagnosis and treatment of cancer. Specific molecular markers are characteristic of particular cancer types. IHC is also widely used in basic research to understand the distribution and localization of biomarkers in different parts of a tissue.

The term "sample", as used herein, refers to a sample obtained from a patient. The sample may be of any biological tissue or fluid. Such samples include, but are not limited to, sputum, blood, serum, plasma, blood cells (e.g., white cells), tissue, core or fine needle biopsy samples, cell-containing body fluids, free floating nucleic acids, urine, peritoneal fluid, and pleural fluid, or cells there from. Biological samples may also include sections of tissues such as frozen or fixed sections taken for histological purposes or microdissected cells or extracellular parts thereof. A biological sample to be analyzed is tissue material from neoplastic lesion taken by aspiration or punctuation, excision or by any other surgical method leading to biopsy or resected cellular material. Such biological sample may comprise cells obtained from a patient. The cells may be found in a cell "smear" collected, for example, by a nipple aspiration, ductal-lavarge, fine needle biopsy or from provoked or spontaneous nipple discharge. In another embodiment, the sample is a body fluid. Such fluids include, for example, blood fluids, serum, plasma, lymph, ascitic fluids, gynecological fluids, or urine but not limited to these fluids.

A "tumor sample" is a sample containing tumor material e.g. tissue material from a neoplastic lesion taken by aspiration or puncture, excision or by any other surgical method leading to biopsy or resected cellular material, including preserved material such as fresh frozen material, formalin fixed material, paraffin embedded material and the like. Such a biological sample may comprise cells obtained from a patient. The cells may be found in a cell "smear" collected, for example, by a nipple aspiration, ductal l'avage, fine needle biopsy or from provoked or spontaneous nipple discharge. In another embodiment, the sample is a body fluid. Such fluids include, for example, blood fluids, serum, plasma, lymph, ascitic fluids, gynecological fluids, or urine but not limited to these fluids.

The term "mathematically combining expression levels", within the meaning of the invention shall be understood as deriving a numeric value from a determined expression level of a gene and applying an algorithm to one or more of such numeric values to obtain a combined numerical value or combined score.

A "score" within the meaning of the invention shall be understood as a numeric value, which is related to the outcome of a patient's disease and/or the response of a tumor to chemotherapy. The numeric value is derived by combining the expression levels of marker genes using pre-specified coefficients in a mathematic algorithm. The expression levels can be employed as CT or delta-CT values obtained by kinetic RT-PCR, as absolute or relative fluorescence intensity values obtained through microarrays or by any other method useful to quantify absolute or relative RNA levels. Combining these expression levels can be accomplished for example by multiplying each expression level with a defined and specified coefficient and summing up such products to yield a score. The score may be also derived from expression levels together with other information, e. g. clinical data like tumor size, lymph node status or tumor grading as such variables can also be coded as numbers in an equation. The score may be used on a continuous scale to predict the response of a tumor to chemotherapy and/or the outcome of a patient's disease. Cut-off values may be applied to distinguish clinical relevant subgroups. Cut-off values for such scores can be determined in the same way as cut-off values for conventional diagnostic markers and are well known to those skilled in the art. A useful way of determining such cut-off value is to construct a receiver-operator curve (ROC curve) on the basis of all conceivable cut-off values, determine the single point on the ROC curve with the closest proximity to the upper feft corner (0/1) in the ROC plot. Obviously, most of the time cut-off values will be determined by less formalized procedures by choosing the combination of sensitivity and specificity determined by such cut-off value providing the most beneficial medical information to the problem investigated.

The term "a PCR based method" as used herein refers to methods comprising a polymerase chain reaction (PCR). This is an approach for exponentially amplifying nucleic acids, like DNA or RNA, via enzymatic replication, without using a living organism. As PCR is an in vitro technique, it can be performed without restrictions on the form of DNA, and it can be extensively modified to perform a wide array of genetic manipulations. When it comes to the determination of expression levels, a PCR based method may for example be used to detect the presence of a given mRNA by (1) reverse transcription of the complete mRNA pool (the so called transcriptome) into cDNA with help of a reverse transcriptase enzyme, and (2) detecting the presence of a given cDNA with help of respective primers. This approach is commonly known as reverse transcriptase PCR (rtPCR). Moreover, PCR-based methods comprise e.g. real time PCR, and, particularly suited for the analysis of expression levels, kinetic or quantitative PCR (qPCR).

A "microarray" herein also refers to a "biochip" or "biological chip". an array of regions having a density of discrete regions of at least about 100/cm$^2$, and preferably at least about 1000/cm$^2$. The regions in a microarray have typical dimensions, e.g., diameters, in the range of between about 10-250 $\mu$m, and are separated from other regions in the array by about the same distance.

The term "hybridization-based method", as used herein, refers to methods imparting a process of combining complementary, single-stranded nucleic acids or nucleotide analogues into a single double stranded molecule. Nucleotides or nucleotide analogues will bind to their complement under normal conditions, so two perfectly complementary strands will bind to each other readily. In bioanalytics, very often labeled, single stranded probes are in order to find complementary target sequences. If such sequences exist in the sample, the probes will hybridize to said sequences which can then be detected due to the label. Other hybridization based methods comprise microarray and/or biochip methods. Therein, probes are immobilized on a solid phase, which is then exposed to a sample. If complementary nucleic acids exist in the sample, these will hybridize to the probes and can thus be detected. These approaches are also known as "array based methods". Yet another hybridization based method is PCR, which is described above. When it comes to the determination of expression levels, hybridization based methods may for example be used to determine the amount of mRNA for a given gene.

The term "marker gene" as used herein, refers to a differentially expressed gene whose expression pattern may be utilized as part of a predictive, prognostic or diagnostic process in malignant neoplasia or cancer evaluation, or which, alternatively, may be used in methods for identifying compounds useful for the treatment or prevention of malignant neoplasia and head and neck, colon or breast cancer in par-ticular. A marker gene may also have the characteristics of a target gene.

An "algorithm" is a process that performs some sequence of operations to produce information.

The term "measurement at a protein level", as used herein, refers to methods which allow the quantitative and/or qualitative determination of one or more proteins in a sample. These methods include, among others, protein purification, including ultracentrifugation, precipitation and chromatography, as well as protein analysis and determination, including immunohistochemistry, immunofluorescence, ELISA (enzyme linked immunoassay), RIA (radioimmunoassay) or the use of protein microarrays, two- hybrid screening, blotting methods including western blot, one- and two dimensional gelelectrophoresis, isoelectric focusing as well as methods being based on mass spectrometry like MALDI-TOF and the like.

The term "kinetic PCR" or "quantitative PCR" (qPCR) refers to any type of a PCR method which allows the quantification of the template in a sample. Quantitative real-time PCR comprise different techniques of performance or product detection as for example the TaqMan technique or the LightCycler technique. The TaqMan technique, for examples, uses a dual-labelled fluorogenic probe. The TaqMan real-time PCR measures accumulation of a product via the fluorophore during the exponential stages of the PCR, rather than at the end point as in conventional PCR. The exponential increase of the product is used to determine the threshold cycle, $C_T$, i.e. the number of PCR cycles at which a significant exponential increase in fluorescence is detected, and which is directly correlated with the number of copies of DNA template present in the reaction. The set up of the reaction is very similar to a conventional PCR, but is carried out in a real-time thermal cycler that allows measurement of fluorescent molecules in the PCR tubes. Different from regular PCR, in TaqMan real-time PCR a probe is added to the reaction, i.e., a single-stranded oligonucleotide complementary to a segment of 20-60 nucleotides within the DNA template and located between the two primers. A fluorescent reporter or fluorophore (e.g., 6-carboxyfluorescein, acronym: FAM, or tetrachlorofluorescin, acronym: TET) and quencher (e.g., tetramethylrhodamine, acronym: TAMRA, of dihydrocyclopyrroloindole tripeptide "minor groove binder", acronym: MGB) are covalently attached to the 5' and 3' ends of the probe , respectively [2]. The close proximity between fluorophore and quencher attached to the probe inhibits fluorescence from the fluorophore. During PCR, as DNA synthesis commences, the 5' to 3' exonuclease activity of the Taq polymerase degrades that proportion of the probe that has annealed to the template (Hence its name: Taq polymerase + PacMan) . Degradation of the probe releases the fluorophore from it and breaks the close proximity to the quencher, thus relieving the quenching effect and allowing fluorescence of the fluorophore. Hence, fluorescence detected in the real-time PCR thermal cycler is directly proportional to the fluorophore released and the amount of DNA template present in the PCR.

"Primer" and "probes", within the meaning of the invention, shall have the ordinary meaning of this term which is well known to the person skilled in the art of molecular biology. In a preferred embodiment of the invention "primer" and "probes" shall be understood as being polynucleotide molecules having a sequence identical, complementary, homologous, or homologous to the complement of regions of a target polynucleotide which is to be detected or quantified. In yet another embodiment nucleotide analogues and/or morpholinos are also comprised for usage as primers and/or probes. "Individually labeled probes", within the meaning of the invention, shall be understood as being molecular probes comprising a polynucleotide, oligonucleotide or nucleotide analogue and a label, helpful in the detection or quantification of the probe. Preferred labels are fluorescent molecules, luminescent molecules, radioactive molecules, enzymatic molecules and/or quenching molecules.

**Object of the Invention**

It is one object of the present invention to provide an improved method for the prediction of a response of a tumor in a patient suffering from or at risk of developing a neoplastic disease - in particular breast cancer - to at least one given mode of treatment.

It is another object of the present invention to avoid unnecessary adjuvant and/or neoadjuvant cytotoxic chemotherapy in patients suffering from a neoplastic disease, especially breast cancer.

It is another object of the present invention to offer a more robust and specific diagnostic assay system than conventional immunohistochemistry for clinical routine fixed tissue samples that better helps the physician to select individualized treatment modalities.

In a more preferred embodiment the disclosed method can be used to select a suitable therapy for a neoplastic disease, particularly breast cancers.

It is another object of the present invention to detect new targets for newly available targeted drugs, or to determine drugs yet to be developed.

**Summary of the Invention**

It must be noted that, as used in the specification and the appended claims, the singular forms "a", "an" and "the" include singular and/or plural referents unless the context clearly dictates otherwise. It is moreover to be understood that, in case parameter ranges are given which are delimited by numeric values, the ranges are deemed to include these limitation values.

The above problems are solved by methods and means provided by the invention.

Estrogen receptor status is generally determined using immunohistochemistry. HER2/NEU (ERBB2) status is generally determined using immunohistochemistry and fluorescence in situ hybridization. However, estrogen receptor status and HER2/NEU (ERBB2) status may, for the purposes of the invention, be determined by any suitable method, e.g. immunohistochemistry, fluorescence in situ hybridization (FISH), or gene expression analysis.

Disclosed is a method for predicting a response to and/or benefit of chemotherapy including neoadjuvant chemotherapy in a patient suffering from or at risk of developing recurrent neoplastic disease, in particular breast cancer. Said method comprises the steps of:

(a) determining in a tumor sample from said patient the gene expression levels of at least 3 of the following 9 genes: UBE2C, BIRCS, RACGAP1, DHCR7, STC2, AZGP1, RBBP8, IL6ST, and MGP

(b) mathematically combining expression level values for the genes of the said set which values were determined in the tumor sample to yield a combined score, wherein said combined score is predicting said response and/or benefit of chemotherapy.

WO 2011/120984A1 utilizes the nine genes, however, for predicting an outcome of breast cancer in an estrogen receptor positive and HER2 negative tumor of a breast cancer patient, which is not related with the method of the present invention which is predicting a response to/or benefit of chemotherapy. The genes of the present invention are used for a different aim.

The method of the invention for predicting a response to and/or benefit of chemotherapy with taxane or anthracycline, including neoadjuvant chemotherapy, in a patient suffering from or at risk of developing recurrent breast cancer comprises the steps of:

(a) determining in a breast tumor sample from said patient the RNA expression levels of the following 8 genes: UBE2C, RACGAP1, DHCR7, STC2, AZGP1, RBBP8, IL6ST, and MGP, indicative of a response to chemotherapy for a tumor or

(b) determining in a breast tumor sample from said patient the RNA expression levels of the following 8 genes: UBE2C, BIRC5, DHCR7, STC2, AZGP1, RBBP8, IL6ST, and MGP; indicative of a response to chemotherapy for a tumor

(c) mathematically combining expression level values for the genes of the said set which values were determined in the tumor sample to yield a combined score, wherein said combined score is predicting said response and/or benefit of chemotherapy.

A further embodiment discloses a method comprising:

(a) determining in a tumor sample from said patient the RNA expression levels of the following 8 genes: UBE2C, BIRCS, DHCR7, STC2, AZGP1, RBBP8, IL6ST, and MGP; indicative of a response to chemotherapy for a tumor **while BIRCS** may be replaced by UBE2C or TOP2A or RACGAP1 or AURKA or NEK2 or E2F8 or PCNA or CYBRD1 or DCN or ADRA2A or SQLE or CXCL12 or EPHX2 or ASPH or PRSS16 or EGFR or CCND1 or TRIM29 or DHCR7 or PIP or TFAP2B or WNT5A or APOD or PTPRT with the proviso that after a replacement 8 different genes are selected; and **while UBE2C** may be replaced by BIRC5 or RACGAP1 or TOP2A or AURKA or NEK2 or E2F8 or PCNA or CYBRD1 or ADRA2A or DCN or SQLE or CCND1 or ASPH or CXCL12 or PIP or PRSS16 or EGFR or DHCR7 or EPHX2 or TRIM29 with the proviso that after a replacement 8 different genes are selected; and

**while DHCR7** may be replaced by AURKA, BIRC5, UBE2C or by any other gene that may replace BIRC5 or UBE2C with the proviso that after a replacement 8 different genes are selected; and

**while STC2** may be replaced by INPP4B or IL6ST or SEC14L2 or MAPT or CHPT1 or ABAT or SCUBE2 or ESR1 or RBBP8 or PGR or PTPRT or HSPA2 or PTGER3 with the proviso that after a replacement 8 different genes are selected; and

**while AZGP1** may be replaced by PIP or EPHX2 or PLAT or SEC14L2 or SCUBE2 or PGR with the proviso that after a replacement 8 different genes are selected; and

**while RBBP8** may be replaced by CELSR2 or PGR or STC2 or ABAT or IL6ST with the proviso that after a replacement 8 different genes are selected; and

**while IL6ST** may be replaced by INPP4B or STC2 or MAPT or SCUBE2 or ABAT or PGR or SEC14L2 or ESR1 or GJA1 or MGP or EPHX2 or RBBP8 or PTPRT or PLAT with the proviso that after a replacement 8 different genes are selected; and

**while MGP** may be replaced by APOD or IL6ST or EGFR with the proviso that after a replacement 8 different genes are selected;

(b) mathematically combining expression level values for the genes of the said set which values were determined in the tumor sample to yield a combined score, wherein said combined score is predicting said response and/or benefit of chemotherapy.

The method of the invention particularly suited for predicting a response to cytotoxic taxane/anthracycline-containing chemotherapy, preferably in Her2/neu negative, estrogen receptor positive (luminal) tumors, preferably in the neodadjuvant mode.

According to an aspect of the invention there is provided a method as described above, wherein said expression level is determined as a mRNA level. According to an aspect of the disclosure there is provided a method as described above, wherein said expression level is determined as a gene expression level.

According to an aspect of the invention there is provided a method as described above, wherein said expression level is determined by at least one of

a PCR based method,
a microarray based method,
a hybridization based method, and.
a sequencing and/or next generation sequencing approach

According to an aspect of the invention there is provided a method as described above, wherein said determination of expression levels is in a formalin-fixed paraffin-embedded tumor sample or in a fresh-frozen tumor sample.

According to an aspect of the invention there is provided a method as described above, wherein the expression level of said at least one marker gene is determined as a pattern of expression relative to at least one reference gene or to a computed average expression value.

According to an aspect of the invention there is provided a method as described above, wherein said step of mathematically combining comprises a step of applying an algorithm to values representative of an expression level of a given gene.

According to an aspect of the invention there is provided a method as described above, wherein said algorithm is a linear combination of said values representative of an expression level of a given gene.

According to an aspect of the invention there is provided a method as described above, wherein a value for a representative of an expression level of a given gene is multiplied with a coefficient.

According to an aspect of the invention there is provided a method as described above, wherein one, two or more thresholds are determined for said combined score and discriminated into high and low risk, high, intermediate and low risk, or more risk groups by applying the threshold on the combined score.

According to an aspect of the invention there is provided a method as described above, wherein a high combined score is indicative of benefit from a more aggressive therapy, e.g. cytotoxic chemotherapy. The skilled person understands that a "high score" in this regard relates to a reference value or cut-off value. The skilled person further understands that depending on the particular algorithm used to obtain the combined score, also a "low" score below a cut off or reference value can be indicative of benefit from a more aggressive therapy, e.g. cytotoxic chemotherapy.

According to an aspect of the invention there is provided a method as described above, wherein information regarding nodal status of the patient is processed in the step of mathematically combining expression level values for the genes to yield a combined score.

According to an aspect of the invention there is provided a method as described above, wherein said information regarding nodal status is a numerical value $\leq 0$ if said nodal status is negative and said information is a numerical value

> 0 if said nodal status positive or unknown. In exemplary embodiments of the invention a negative nodal status is assigned the value 0, an unknown nodal status is assigned the value 0.5 and a positive nodal status is assigned the value 1. Other values may be chosen to reflect a different weighting of the nodal status within an algorithm.

According to an aspect of the invention there is provided a method as described above, wherein said information regarding tumor size of the patient is processed in the step of mathematically combining expression level values for the genes to yield a combined score.

According to an aspect of the invention there is provided a method as described above, wherein said information regarding nodal status and tumor size of the patient is processed in the step of mathematically combining expression level values for the genes to yield a combined score.

The invention further relates to the use of a kit for performing the method as described above, said kit comprising a set of oligonucleotides capable of specifically binding sequences or to sequences of fragments of the genes in a combination of genes, wherein

(i) said combination comprises at.least the 8 genes UBE2C, BIRCS, DHCR7, STC2, AZGP1, RBBP8, IL6ST, and MGP; or

(ii) said combination comprises at least the 8 genes UBE2C, RACGAP1, DHCR7, STC2, AZGP1, RBBP8, IL6ST, and MGP.

Further a computer program product is disclosed which is capable of processing values representative of an expression level of a combination of genes mathematically combining said values to yield a combined score, wherein said combined score is predicting said response and/or benefit of chemotherapy of said patient.

Said computer program product may be stored on a data carrier or implemented on a diagnostic system capable of outputting values representative of an expression level of a given gene, such as a real time PCR system.

If the computer program product is stored on a data carrier or running on a computer, operating personal can input the expression values obtained for the expression level of the respective genes. The computer program product can then apply an algorithm to produce a combined score indicative of benefit from cytotoxic chemotherapy for a given patient.

The methods of the present invention have the advantage of providing a reliable prediction of response and/or benefit of chemotherapy based on the use of only a small number of genes. The methods of the present invention have been found to be especially suited for analyzing the response and/or benefit of chemotherapy of patients with tumors classified as ESR1 positive and ERBB2 negative.

**Detailed description of the invention**

Additional details, features, characteristics and advantages of the object of the invention are disclosed in the sub-claims, and the following description of the respective figures and examples, which, in an exemplary fashion, show preferred embodiments of the present invention.

Four public available gene expression data sets (Affymetrix HG-U133A) were retrieved from the gene expression omnibus (GEO) data repository. All analyzed breast cancer patients were treated with anthracycline or taxan/anthracyciine-based neoadjuvant chemotherapy. Microarray cell files were MAS5 normalized with a global scaling procedure and a target intensity of 500. Pathological complete response (pCR) was used as the primary endpoint for the assessment of treatment response. The analysis was performed in all HER2/neu-negative breast cancer patients and in the subset of ER-positive, HER2-negative breast cancer patients according to pre-specified cut-off levels (ERBB2 probeset 216836 < 6000 = HER2/neu-negative, ERBB2 probeset 216836 < 6000 and ESR1 probeset > 1000 = ER-positive/HER2/neu-negative).

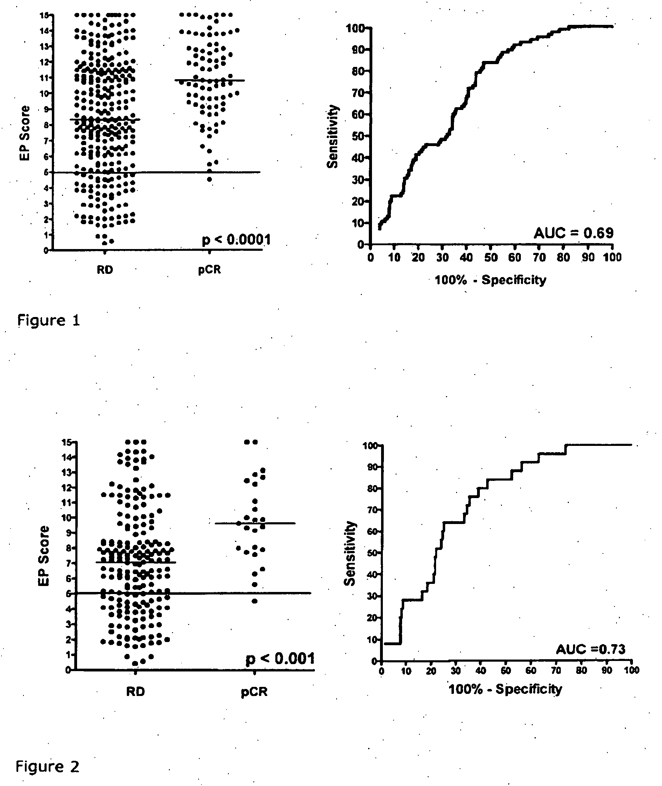

The T5 score was examined in 374 HER2-negative breast cancer patients treated with neoadjuvant therapy (figure 1). Among the 374 patients, 63 tumors (16.8 %) were classified as T5-low-risk, whereas 311 tumors (83.2%) were TS-high-risk. Only one of the T5-low-risk tumors achieved a pCR after neoadjuvant therapy, whereas 84 of the 85 pCR events were classified as T5-high risk. The sensitivity of the T5 score was 99% and the negative predictive value 98% with an area under the receiver operating characteristic curve of 0.69(figure 1).

The Figure 1 shows:

a) T5 score distribution in 374 HER2/neu-negative breast cancer patients (85 pCR events vs. 289 samples with residual disease); two-sided Mann-Whitney Test

b) Using the pre-specified cut-off T5 score 5, the sensitivity was 99%, the specificity 21%, the negative predictive value 98% and the positive predictive value 27% with an area under the receiver operating curve of 0.69.

The T5 score was examined in 221 ER-positive, HER2-negative breast cancer patients treated with neoadjuvant therapy (figure 2). Among the 221 patients, 61 tumors (27.6 %) were classified as T5-low-risk, whereas 160 tumors (72.4%) were T5-high-risk. Only one of the T5-low-risk tumors achieved a pCR after neoadjuvant therapy, whereas 24 of the 25 pCR events were classified as T5-high risk. The sensitivity of the T5 score was 96% and the negative predictive value 98% with an area under the receiver operating characteristic curve of 0.73 (figure 2).
The Figure 2 shows:

c) T5 score distribution in 221 estrogen receptor positive and HER2/neu-negative breast cancer patients (25 pCR events vs. 196 samples with residual disease); two-sided Mann-Whitney Test

d) Using the pre-specified cut-off TS score 5, the sensitivity was 96%, the specificity 30%, the negative predictive value 98% and the positive predictive value 15% with an area under the receiver operating curve of 0.73.

Herein disclosed are unique combinations of marker genes which can be combined into an algorithms for the here presented new predictive test. Technically, the method of the invention can be practiced using two technologies: 1.) Isolation of total RNA from fresh or fixed tumor tissue and 2.) Quantitative RT-PCR of the isolated nucleic acids. Alternatively, it is contemplated to measure expression levels using alternative technologies, e.g by microarray, in particular affymetrix U-133 arrays or by measurement at a protein level.
The methods of the invention are based on quantitative determination of RNA species isolated from the tumor in order to obtain expression values and subsequent bioinformatic analysis of said determined expression values. RNA species can be isolated from any type of tumor sample, e.g. biopsy samples, smear samples, resected tumor material, fresh frozen tumor tissue or from paraffin embedded and formalin fixed tumor tissue. First, RNA levels of genes coding for specific combinations of the genes UBE2C, BIRC5, DHCR7, RACGAP1, AURKA, PVALB, NMU, STC2, AZGP1, RBBP8, IL6ST, MGP, PTGER3, CXCL12, ABAT, CDH1, and PIP or specific combinations thereof, as indicated, are determined. Based on these expression values a predictive score is calculated by a mathematical combination, e.g. according to formulas T5, T1, T4, or T5b (see below).
A high score value indicates an increased likelihood of a pathological complete response after neoadjuvant chemotherapy treatment, a low score value indicates a decreased likelihood of developing a pathological complete response after neoadjuvant treatment. Consequently, a high score also indicates that the patient is a high risk patient who will benefit from a more aggressive therapy, e.g. cytotoxic chemotherapy.
Table 1, below, shows the combinations of genes used for each algorithm.

Table 1: Combination of genes for the respective algorithms:

| Gene | Algo_T1 | Algo_T4 | Alg_T5 | Algo_T5b |
|---|---|---|---|---|
| UBE2C | | | X | X |
| BIRC5 | X | X | X | |
| DHCR7 | | X | X | X |
| RACGAP1 | | X | | X |
| AURKA | X | | | |
| PVALB | X | X | | |
| NMU | X | | | X |
| STC2 | X | X | X | |
| AZGP1 | | | X | X |
| RBBP8 | X | | X | X |
| IL6ST | | X | X | X |
| MGP | | | X | X |
| PTGER3 | X | X | | |
| CXCL12 | X | X | | |
| ABAT | | X | | |
| CDH1 | X | | | |

(continued)

| Gene | Algo_T1 | Algo_T4 | Alg_T5 | Algo_T5b |
|------|---------|---------|--------|----------|
| PIP | X | | | |

Table 2, below, shows Affy probeset ID and TaqMan design ID mapping of the marker genes of the present invention.

Table 2: Gene symbol, Affy probeset ID and TaqMan design ID mapping:

| Gene | Design ID | Probeset ID |
|------|-----------|-------------|
| UBE2C | R65 | 202954_at |
| BIRC5 | SC089 | 202095_s_at |
| DHCR7 | CAGMC3344 | 201791_s_at |
| RACGAP1 | R125-2 | 222077_s_at |
| AURKA | CAGMC336 | 204092_s_at |
| PVALB | CAGMC339 | 205336_at |
| NMU | CAGMC331 | 206023_at |
| STC2 | R52 | 203438_at |
| AZGP1 | CAGMC372 | 209309_at |
| RBBP8 | CAGMC347 | 203344_s_at |
| IL6ST | CAGMC312 | 212196_at |
| MGP | CAGMC383 | 202291_s_at |
| PTGER3 | CAGMC315 | 213933_at |
| CXCL12 | CAGMC342 | 209687_at |
| ABAT | CAGMC338 | 209460_at |
| CDH1 | CAGMC335 | 201131_s_at |

Table 3, below, shows full names, Entrez GeneID, gene bank accession number and chromosomal location of the marker genes of the present invention

| Official Symbol | Official Full Name | Entrez GeneID | Accesion Number | Location |
|-----------------|--------------------|---------------|-----------------|----------|
| UBE2C | ubiquitin-conjugating enzyme E2C | 11065 | U73379 | 20q13. 12 |
| BIRC5 | baculoviral IAP re-peat-containing 5 | 332 | U75285 | 17q25 |
| DHCR7 | 7-dehydrocholesterol reductase | 1717 | AF034544 | 11q13. 4 |
| STC2 | staniocalcin 2 | 8614 | AB012664 | 5q35.2 |
| RBBP8 | retinoblastoma binding protein 8 | 5932 | AF043431 | 18q11. 2 |
| IL6ST | interleukin 6 signal transducer | 3572 | M57230 | 5q11 |
| MGP | matrix Gla protein | 4256 | M58549 | 12p12. 3 |
| AZGP1 | alpha-2-glycoprotein 1, zinc-binding | 563 | BC005306 | 11q22. 1 |
| RACGAP1 | Rac GTPase activating protein 1 | 29127 | NM_013277 | 12q13 |
| AURKA | aurora kinase A | 6790 | BC001280 | 20q13 |
| PVALB | parvalbumin | 5816 | NM_002854 | 22q13. 1 |
| NMU | neuromedin U | 10874 | X76029 | 4q12 |
| PTGER3 | prostaglandin E receptor 3 (subtype EP3) | 5733 | X83863 | 1p31.2 |
| CXCL12 | chemokine (C-X-C motif) ligand 12 (stromal cell-derived factor 1) | 6387 | L36033 | 10q11. 1 |
| ABAT | 4-aminobutyrat aminotransferase | 18 | L32961 | 16p13. 2 |

(continued)

| Official Symbol | Official Full Name | Entrez GeneID | Accesion Number | Location |
|---|---|---|---|---|
| CDH1 | cadherin 1, type 1, E-cadherin (epithelial) | 999 | L08599 | 16q22. 1 |
| PIP | prolactin-induced protein | 5304 | NMM_002652 | 7q32-qter |

Example algorithm T5:

Algorithm T5 is a committee of four members where each member is a linear combination of two genes. The mathematical formulas for T5 are shown below; the notation is the same as for T1. T5 can be calculated from gene expression data only.

$$riskMember1 = 0.434039 \ [0.301..0.567] * (0.939 * BIRC5 -3.831)$$

$$-0.491845 \ [-0.714..-0.270] * (0.707 * RBBP8 -0.934)$$

$$riskMember2 = 0.488785 \ [0.302..0.675] * (0.794 * UBE2C -1.416)$$

$$-0.374702 \ [-0.570..-0.179] * (0.814 * IL6ST -5.034)$$

$$riskMember3 = -0.39169 \ [-0.541..-0.242] * (0.674 * AZGP1 -0.777)$$

$$+0.44229 \ [0.256..0.628] * (0.891 * DHCR7 -4.378)$$

$$riskMember4 = -0.377752 \ [-0.543..-0.212] * (0.485 * MGP +4.330)$$

$$-0.177669 \ [-0.267..-0.088] * (0.826 * STC2 -3.630)$$

$$risk = riskMember1 + riskMember2 + riskMember3 + riskMember4$$

Coefficients on the left of each line were calculated as COX proportional hazards regression coefficients, the numbers in squared brackets denote 95% confidence bounds for these coefficients. In other words, instead of multiplying the term (0.939 * BIRC5 -3.831) with 0.434039, it may be multiplied with any coefficient between 0.301 and 0.567 and still give a predictive result with in the 95% confidence bounds. Terms in round brackets on the right of each line denote a platform transfer from PCR to Affymetrix: The variables PVALB, CDH1, ... denote PCR-based expressions normalized by the reference genes (delta-Ct values), the whole term within round brackets corresponds to the logarithm (base 2) of Affymetrix microarray expression values of corresponding probe sets.

Example algorithm T5clin:

Algorithm T5clin is a combined score consisting of the T5 score and clinical parameters (nodal status and tumor size).

$$T5clin = 0.35 * t + 0.64 * n + 0.28*s$$

where t codes for tumor size (1: ≤1 cm, 2: >1 cm to ≤2 cm, 3: >2 cm to ≤5 cm, 4: >5 cm), and $n$ for nodal status (1: negative, 2: 1 to 3 positive nodes, 3: 4 to 10 positive nodes, 4: > 10 positive nodes).
In a preferred in embodiment, the threshold for the T5clin score is 3.3.

Example algorithm T1:

Algorithm T1 is a committee of three members where each member is a linear combination of up to four variables. In general variables may be gene expressions or clinical variables. In T1 the only non-gene variable is the nodal status coded 0, if patient is lymph-node negative and 1, if patient is lymph-node-positive. The mathematical formulas for T1 are shown below.

$$riskMember1 = +0.193935 [0.108..0.280] * (0.792 * PVALB -2.189)$$
$$-0.240252 [-0.400..-0.080] * (0.859 * CDH1 -2.900)$$
$$-0.270069 [-0.385..-0.155] * (0.821 * STC2 -3.529)$$
$$+1.2053 [0.534..1.877] * nodalStatus$$

$$riskMember2 = -0.25051 [-0.437..-0.064] * (0.558 * CXCL12 +0.324)$$
$$-0.421992 [-0.687..-0.157] * (0.715 * RBBP8 -1.063)$$
$$+0.148497 [0.029..0.268] * (1.823 * NMU -12.563)$$
$$+0.293563 [0.108..0.479] * (0.989 * BIRC5 -4.536)$$

$$riskMember3 = +0.308391 [0.074..0.543] * (0.812 * AURKA -2.656)$$
$$-0.225358 [-0.395..-0.055] * (0.637 * PTGER3 + 0.492)$$
$$-0.116312 [-0.202..-0.031] * (0.724 * PIP + 0.985)$$

$$risk = + riskMember1 + riskMember2 + riskMember3$$

Coefficients on the left of each line were calculated as COX proportional hazards regression coefficients, the numbers in squared brackets denote 95% confidence bounds for these coefficients. Terms in round brackets on the right of each line denote a platform transfer from PCR to Affymetrix: The variables PVALB, CDH1, ... denote PCR-based expressions normalized by the reference genes, the whole term within round brackets corresponds to the logarithm (base 2) of Affymetrix microarray expression values of corresponding probe sets.

Example algorithm T4:

Algorithm T4 is a linear combination of motifs. The top 10 genes of several analyses of Affymetrix datasets and PCR data were clustered to motifs. Genes not belonging to a cluster were used as single gene-motifs. COX proportional hazards regression coefficients were found in a multivariate analysis.
In general motifs may be single gene expressions or mean gene expressions of correlated genes. The mathematical formulas for T4 are shown below.

$$prolif = ((0.84 [0.697..0.977] * RACGAP1 -2.174) + (0.85 [0.713..0.988]$$
$$*DHCR7 -3.808)+ (0.94 [0.786..1.089] * BIRC5 -3.734)) / 3$$

$$motiv2 = ((0.83 [0.693..0.96] * IL6ST -5.295) + (1.11 [0.930..1.288] * ABAT$$
$$-7.019) + (0.84 [0.701..0.972] * STC2 -3.857)) / 3$$

$$ptger3 = (PTGER3 * 0.57 [0.475..0.659] + 1.436)$$

$$cxcl12 = (CXCL12 * 0.53 [0.446..0.618] + 0.847)$$

$$pvalb = (PVALB * 0.67 [0.558..0.774] -0.466)$$

Factors and offsets for each gene denote a platform transfer from PCR to Affymetrix: The variables RACGAP1, DHCR7, ... denote PCR-based expressions normalized by CALM2 and PPIA, the whole term within round brackets corresponds to the logarithm (base 2) of Affymetrix microarray expression values of corresponding probe sets.

The numbers in squared brackets denote 95% confidence bounds for these factors.

As the algorithm performed even better in combination with a clinical variable the nodal status was added. In T4 the nodal status is coded 0, if patient is lymph-node negative and 1, if patient is lymph-node-positive. With this, algorithm T4 is:

$$risk = -0.32 [-0.510..-0.137] * motiv2$$
$$+ 0.65 [0.411..0.886] * prolif$$
$$- 0.24 [-0.398..-0.08] * ptger3$$
$$- 0.05 [-0.225..0.131] * cxcl12$$
$$+ 0.09 [0.019..0.154] * pvalb$$
$$+ nodalStatus$$

Coefficients of the risk were calculated as COX proportional hazards regression coefficients, the numbers in squared brackets denote 95% confidence bounds for these coefficients.

Algorithm T5b is a committee of two members where each member is a linear combination of four genes. The mathematical formulas for T5b are shown below, the notation is the same as for T1 and T5. In T5b a non-gene variable is the nodal status coded 0, if patient is lymph-node negative and 1, if patient is lymph-node-positive and 0.5 if the lymph-node status is unknown. T5b is defined by:

$$riskMember1 = 0.359536 [0.153..0.566] * (0.891 * DHCR7 -4.378)$$
$$-0.288119 [-0.463..-0.113] * (0.485 * MGP + 4.330)$$
$$+0.257341 [0.112..0.403] * (1.118 * NMU -5.128)$$
$$-0.337663 [-0.499..-0.176] * (0.674 * AZGP1 -0.777)$$

$$riskMember2 = -0.374940 [-0.611..-0.139] * (0.707 * RBBP8 -0.934)$$
$$-0.387371 [-0.597..-0.178] * (0.814 * IL6ST -5.034)$$
$$+0.800745 [0.551..1.051] * (0.860 * RACGAP1 -2.518)$$
$$+0.770650 [0.323..1.219] * Nodalstatus$$

$$risk = riskMember1 + riskMember2$$

The skilled person understands that these algorithms represent particular examples and that based on the information regarding association of gene expression with the prediction of therapeutic response.

Algorithm Simplification by employing Subsets of Genes

"Example algorithm T5" is a committee predictor consisting of 4 members with 2 genes of interest each. Each member is an independent and self-contained predictor of distant recurrence and/or therapy response, each additional member contributes to robustness and predictive power of the algorithm. The equation below shows the "Example Algorithm T5"; for ease of reading the number of digits after the decimal point has been truncated to 2; the range in square brackets lists the estimated range of the coefficients (mean +/- 3 standard deviations).

T5 Algorithm:

$$+0.41 [0.21..0.61] * BIRC5 -0.33 [-0.57..-0.09] * RBBP8$$

$$+0.38 [0.15..0.61] * UBE2C -0.30 [-0.55..-0.06] * IL6ST$$

$$-0.28 [-0.43..-0.12] * AZGP1 +0.42 [0.16..0.68] * DHCR7$$

$$-0.18 [-0.31..-0.06] * MGP -0.13 [-0.25..-0.02] * STC2$$

c-indies: trainSet=0.724,

Gene names in the algorithm denote the difference of the mRNA expression of the gene compared to one or more housekeeping genes as described above.

Analyzing a cohort different from the finding cohort (234 tumor samples) it was surprising to learn that some simplifications of the "original T5 Algorithm" still yielded a diagnostic performance not significantly inferior to the original TS algorithm. The most straightforward simplification was reducing the committee predictor to one member only. Examples for the performance of the "one-member committees" are shown below:

member 1 only:

$$+0.41 [0.21..0.61] * BIRC5 -0.33 [-0.57..-0.09] * RBBP8$$

c-indices: trainSet=0.653, independentCohort=0.681
member 2 only:

$$+0.38 [0.15..0.61] * UBE2C -0.30 [-0.55..-0.06] * IL6ST$$

c-indices: trainSet=0.664, independentCohort=0.696
member 3 only:

$$-0.28 [-0.43..-0.12] * AZGP1 +0.42 [0.16..0.68] * DHCR7$$

c-indices: trainSet=0.666, independentCohort=0.601
member 4 only:

$$-0.18 [-0.31..-0.06] * MGP -0.13 [-0.25..-0.02] * STC2$$

c-indices: trainSet=0.668, independentCohort=0.593

The performance of the one member committees as shown in an independent cohort of 234 samples is notably reduced compared to the performance of the full algorithm.

Gradually combining more than one but less than four members to a new prognostic committee predictor algorithm, frequently leads to a small but significant increase in the diagnostic performance compared to a one-member committee. It was surprising to learn that there were marked improvements by some combination of committee members while other combinations yielded next to no improvement. Initially, the hypothesis was that a combination of members representing similar biological motives as reflected by the employed genes yielded a smaller improvement than combining members reflecting distinctly different biological motives. Still, this was not the case. No rule could be identified to foretell the combination of some genes to generate an algorithm exhibiting more prognostic power than another combination of genes. Promising combinations could only be selected based on experimental data. Identified combinations of combined committee members to yield simplified yet powerful algorithms are shown below.

members 1 and 2 only:

$$+0.41 [0.21..0.61] * BIRC5 -0.33 [-0.57..-0.09] * RBBP8$$

$$+0.38 [0.15..0.61] * UBE2C -0.30 [-0.55..-0.06] * IL6ST$$

c-indices: trainSet=0.675, independentCohort=0.712

members 1 and 3 only:

$$+0.41 [0.21..0.61] * BIRC5 -0.33 [-0.57..-0.09] * RBBP8$$

$$-0.28 [-0.43..-0.12] * AZGP1 +0.42 [0.16..0.68] * DHCR7$$

c-indices: trainSet=0.697, independentCohort=0.688

members 1 and 4 only:

$$+0.41 [0.21..0.61] * BIRC5 -0.33 [-0.57..-0.09] * RBBP8$$

$$-0.18 [-0.31..-0.06] * MGP -0.13 [-0.25..-0.02] * STC2$$

c-indices: trainSet=0.705, independentCohort=0.679

members 2 and 3 only:

$$+0.38 [0.15..0.61] * UBE2C -0.30 [-0.55..-0.06] * IL6ST$$

$$-0.28 [-0.43..-0.12] * AZGP1 +0.42 [0.16..0.68] * DHCR7$$

c-indices: trainSet=0.698, independentCohort=0.670

members 1, 2 and 3 only:

$$+0.41 [0.21..0.61] * BIRC5 -0.33 [-0.57..-0.09] * RBBP8$$

$$+0.38 [0.15..0.61] * UBE2C -0.30 [-0.55..-0.06] * IL6ST$$

$$-0.28 [-0.43..-0.12] * AZGP1 +0.42 [0.16..0.68] * DHCR7$$

c-indices: trainSet=0.701, independentCohort=0.715

Not omitting complete committee members but a single gene or genes from different committee members is also possible but requires a retraining of the entire algorithm. Still, it can also be advantageous to perform. The performance of simplified algorithms generated by omitting entire members or individual genes is largely identical.

Algorithm Variants by Gene Replacement

Described algorithms, such as "Example algorithm T5", above can be also be modified by replacing one or more genes by one or more other genes. The purpose of such modifications is to replace genes difficult to measure on a specific platform by a gene more straightforward to assay on this platform. While such transfer may not necessarily yield an improved performance compared to a starting algorithm, it can yield the clue to implanting the prognostic algorithm to a particular diagnostic platform. In general, replacing one gene by another gene while preserving the diagnostic power of the predictive algorithm can be best accomplished by replacing one gene by a co-expressed gene with a high correlation (shown e.g. by the Pearson correlation coefficient). Still, one has to keep in mind that the mRNA expression of two genes highly correlative on one platform may appear quite independent from each other when assessed on another platform. Accordingly, such an apparently easy replacement when reduced to practice experimentally may yield disappointingly poor results as well as surprising strong results, always depending on the imponderabilia of the platform employed. By repeating this procedure one can replace several genes.

The efficiency of such an approach can be demonstrated by evaluating the predictive performance of the T5 algorithm score and its variants on the validation cohorts. The following table shows the c-index with respect to endpoint distant recurrence in two validation cohorts.

| Variant | Validation Study A | Validation Study B |
|---|---|---|
| original algorithm T5 | c-index = 0.718 | c-index = 0.686 |
| omission of BIRC5 (setting expression to some constant) | c-index = 0.672 | c-index = 0.643 |
| replacing BIRC5 by UBE2C (no adjustment of the coefficient) | c-index = 0.707 | c-index = 0.678 |

One can see that omission of one of the T5 genes, here shown for BIRC5 for example, notably reduces the predictive performance. Replacing it with another gene yields about the same performance.

A better method of replacing a gene is to re-train the algorithm. Since T5 consists of four independent committee members one has to re-train only the member that contains the replaced gene. The following equations demonstrate replacements of genes of the T5 algorithm shown above trained in a cohort of 234 breast cancer patients. Only one member is shown below, for c-index calculation the remaining members were used unchanged from the original T5 Algorithm. The range in square brackets lists the estimated range of the coefficients: mean +/- 3 standard deviations.

Member 1 of T5:

Original member 1:

$$+0.41\ [0.21..0.61] * BIRC5\ -0.33\ [-0.57..-0.09] * RBBP8$$

c-indices: trainSet==0.724, independentCohort=0.705
replace BIRC5 by TOP2A in member 1:

$$+0.47\ [0.24..0.69] * TOP2A\ -0.34\ [-0.58..-0.10] * RBBP8$$

c-indices: trainSet=0.734, independentCohort=0.694
replace BIRC5 by RACGAP1 in member 1:

$$+0.69\ [0.37..1.00] * RACGAP1\ -0.33\ [-0.57..-0.09] * RBBP8$$

c-indices: trainSet=0.736, independentCohort=0.743
replace RBBP8 by CELSR2 in member 1:

$$+0.38\ [0.19..0.57] * BIRC5\ -0.18\ [-0.41..0.05] * CELSR2$$

c-indices: trainSet=0.726, independentCohort=0.680
replace RBBP8 by PGR in member 1:

$$+0.35\ [0.15..0.54] * BIRC5\ -0.09\ [-0.23..0.05] * PGR$$

c-indices: trainSet=0.727, independentCohort=0.731

Member 2 of T5:

Original member 2:

$$+0.38\ [0.15..0.61] * UBE2C\ -0.30\ [-0.55..-0.06] * IL6ST$$

c-indices: trainSet=0.724, independentCohort=0.725
replace UBE2C by RACGAP1 in member 2:

$$+0.65\ [0.33..0.96] * RACGAP1\ -0.38\ [-0.62..-0.13] * IL6ST$$

c-indices: trainSet=0.735, independentCohort=0.718
replace UBE2C by TOP2A in member 2:

$$+0.42\ [0.20..0.65] * TOP2A\ -0.38\ [-0.62..-0.13] * IL6ST$$

c-indices: trainSet=0.734, independentCohort=0.700
replace IL6ST by INPP4B in member 2:

$$+0.40\ [0.17..0.62] * UBE2C\ -0.25\ [-0.55..0.05] * INPP4B$$

c-indices: trainSet=0.725, independentCohort=0.686
replace IL6ST by MAPT in member 2:

$$+0.45\ [0.22..0.69] * UBE2C\ -0.14\ [-0.28..0.01] * MAPT$$

c-indices: trainSet=0.727, independentCohort=0.711

Member 3 of T5:

Original member 3:

$$-0.28\ [-0.43..-0.12] * AZGP1\ +0.42\ [0.16..0.68] * DHCR7$$

c-indices: trainSet=0.724, independentCohort=0.705
replace AZGP1 by PIP in member 3:

$$-0.10\ [-0.18..-0.02] * PIP\ +0.43\ [0.16..0.70] * DHCR7$$

c-indices: trainSet=0.725, independentCohort=0.692
replace AZGP1 by EPHX2 in member 3:

$$-0.23\ [-0.43..-0.02] * EPHX2\ +0.37\ [0.10..0.64] * DHCR7$$

c-indices: trainSet=0.719, independentCohort=0.698
replace AZGP1 by PLAT in member 3:

$$-0.23\ [-0.40..-0.06] * PLAT\ +0.43\ [0.18..0.68] * DHCR7$$

c-indices: trainSet=0.712, independentCohort=0.715

replace DHCR7 by AURKA in member 3:

$$-0.23\ [-0.39..-0.06]\ *\ AZGP1\ +0.34\ [0.10..0.58]\ *\ AURKA$$

c-indices: trainSet=0.716, independentCohort=0.733

Member 4 of T5:

Original member 4:

$$-0.18\ [-0.31..-0.06]\ *\ MGP\ -0.13\ [-0.25..-0.02]\ *\ STC2$$

c-indices: trainSet=0.724, independentCohort=0.705
replace MGP by APOD in member 4:

$$-0.16\ [-0.30..-0.03]\ *\ APOD\ -0.14\ [-0.26..-0.03]\ *\ STC2$$

c-indices: trainSet=0.717, independentCohort=0.679
replace MGP by EGFR in member 4:

$$-0.21\ [-0.37..-0.05]\ *\ EGFR\ -0.14\ [-0.26..-0.03]\ *\ STC2$$

c-indices: trainSet=0.715, independentCohort=0.708
replace STC2 by INPP4B in member 4:

$$-0.18\ [-0.30..-0.05]\ *\ MGP\ -0.22\ [-0.53..0.08]\ *\ INPP4B$$

c-indices: trainSet=0.719, independentCohort=0.693
replace STC2 by SEC14L2 in member 4:

$$-0.18\ [-0.31..-0.06]\ *\ MGP\ -0.27\ [-0.49..-0.06]\ *\ SEC14L2$$

c-indices: trainSet=0.718, independentCohort=0.681

One can see that replacements of single genes experimentally identified for a quantification with quantitative PCR normally affect the predictive performance of the T5 algorithm, assessed by the c-index only insignificantly.

The following table shows potential replacement gene candidates for the genes of T5 algorithm. Each gene candidate is shown in one table cell: The gene name is followed by the bracketed absolute Pearson correlation coefficient of the expression of the original gene in the TS Algorithm and the replacement candidate, and the HG-U133A probe set ID.

| STC2 | MGP | DHCR7 | AZGP1 | IL6ST | UBE2C | RBBP8 | BIRCS |
|---|---|---|---|---|---|---|---|
| INPP4B (0.500), 205376_at | APOD (0.368), 201525_at | AURKA(0.345), 204092_s_at | PIP (0.530), 206509_at | INPP4B (0.477), 205376_at | BIRCS (0.775), 202095_s_at | CELSR2 (0.548), 204029_at | UBE2C (0.775), 202954_at |
| IL6ST (0.450), 212196_at | IL6ST (0.327), 212196_at | BIRCS (0.323), 202095_s_at | EPHX2 (0.369), 209368_at | STC2 (0.450), 203438_at | RACGAP1 (0.756), | PGR (0.392), 208305_at | TOP2A (0.757), 201292_at |
| SEC14L2 (0.417), 204541_at | EGFR (0.308), 201983_s_at | UBE2C(0.315), 202954_at | PLAT (0.366), 201860_s_at | MAPT (0.440), 206401_s_at | TOP2A (0.753), 201292_at | STC2 (0.361), 203438_at | RACGAP1 (0.704), |
| MAPT (0.414), 206401_s_at | | | SEC14L2 (0.351), 204541_at | SCUBE2 (0.418), 219197_s_at | AURKA (0.694), 204092_s_at | ABAT (0.317), 209459_s_at | AURKA (0.681), 204092_s_at |
| CHPT1 (0.410), 221675_s_at | | | SCUBE2 (0.331), 219197_s_at | ABAT (0.389), 209459_s_at | NEK2 (0.684), 204026_s_at | IL6ST (0.311), 212196_at | NEK2 (0.680), 204026_s_at |
| ABAT (0.409), 209459_s_at | | | PGR (0.302), 208305_at | PGR (0.377), 208305_at | E2F8 (0.652), 219990_at | | E2F8 (0.640), 219990_at 201202_at |
| SCUBE2 (0.406), 219197_s_at | | | | SEC14L2 (0.356), 204541_at | PCNA (0.589), 201202_at | | CYBRD1 (0.462), 217889_s_at |
| ESR1 (0.394), 205225_at | | | | ESR1 (0.353), 205225_at | CYBRD1 (0.486), 217889_s_at | | DCN (0.439), 209335_at |
| RBBP8 (0.361), 203344_s_at | | | | GJA1 (0.335), 201667_at | AORA2A (0.391), 209869_at | | ADRA2A (0.416), 209869_at |
| PGR (0.347), 208305_at | | | | MGP (0.327), 202291_s_at | DCN (0.384), 209335_at | | SQLE (0.415), 209218_at |
| PTPRT (0.343), 205948_at | | | | EPHX2 (0.313), 209368_at | SQLE (0.369), 209218_at | | CXCL12 (0.388), 209687_at |
| HSPA2 (0.317), 211538_s_at | | | | R88P8 (0.311), 203344_s_at | CCND1 (0.347), 208712_at | | EPHX2 (0.362), 209368_at |
| PTGER3 (0.314), 210832_x_at | | | | PTPRT (0.303), 205948_at | ASPH (0.344), 210896_s_at | | ASPH (0.352), 210896_s_at |
| | | | | PLAT (0.301), 201860_s_at | CXCL12 (0.342), 209687_at | | PRSS16 (0.352), 208165_s_at |
| | | | | | PIP (0.328), 206509_at | | |

**20**

| BIRCS | RBBP8 | UBE2C | IL6ST | AZGP1 | DHCR7 | MGP | STC2 |
|---|---|---|---|---|---|---|---|
| EGFR (0.346), 201983_s_at | | PRSS16 (0.326), 208165_s_at | | | | | |
| CCND1 (0.331), 208712_at | | EGFR (0.320), 201983_s_at | | | | | |
| TRIM29 (0.325), 202504_at | | DHCR7 (0.315), 201791_s_at | | | | | |
| DHCR7 (0.323), 201791_s_at | | EPHX2 (0.315), 209368_at | | | | | |
| PIP (0.308), 206509_at | | TRIM29 (0.311), 202504_at | | | | | |
| TFAP28 (0.306), 214451_at | | | | | | | |
| WNTSA (0.303), 205990_s_at | | | | | | | |
| APOD (0.301), 201525_at | | | | | | | |
| PTPRT (0.301), 205948_at | | | | | | | |

The sequences of the primers and probes were as follows:

Table 1 Primer and probe sequences for the respective genes:

| gene | probe | Seq ID | forward primer | Seq ID | reverse primer | Seq ID |
|---|---|---|---|---|---|---|
| ABAT | TCGCCCTAAGAGGCTCT-TCCTC | 1 | GGCAACTTGAGGTCT-GACTTTTG | 2 | GGTCAGCTCACAAGTGGT-GTGA | 3 |
| ADRA2A | TTGTCCTTTCCCCCCTC-CGTGC | 4 | CCCCAAGAGCTGTTAGG-TATCAA | 5 | TCAATGACATGATCT-CAACCAGAA | 6 |
| APOD | CATCAGCTCTCAACTCCT-GGTTAACA | 7 | ACTCACTAATGGAAAACG-GAAAGATC | 8 | TCACCTTCGATTTGAT-TCACAGTT | 9 |
| ASPH | TGGGAGGAAGGCAAGGT-GCTCATC | 10 | TGTGCCAACGAGACCAA-GAC | 11 | TCGTGCTCAAAGGAGT-CATCA | 12 |
| AURKA | CCGTCAGCCTGTGCTAG-GCAT | 13 | AATCTGGAGGCAAGGTTC-GA | 14 | TCTGGATTTGCCTCCTGT-GAA | 15 |
| BIRC5 | AGCCAGATGACGAC-CCCATAGAGGAACA | 16 | CCCAGTGTTTCTTCTGCT-TCAAG | 17 | CAACCGGACGAAT-GCTTTT | 18 |
| CELSR2 | ACTGACTTTCCTTCT-GGAGCAGGTGGC | 19 | TCCAAGCATGTATTCCA-GACTTGT | 20 | TGCCCACAGCCTCTTTT-TCT | 21 |
| CHPT1 | CCACGGCCACCGAAGAG-GCAC | 22 | CGCTCGTGCTCATCTC-CTACT | 23 | CCCAGTGCACATAAAAGG-TATGTC | 24 |
| CXCL12 | CCACAGCAGGGTTTCAG-GTTCC | 25 | GCCACTACCCCCTCCT-GAA | 26 | TCACCTTGCCAACAGT-TCTGAT | 27 |
| CYBRD1 | AGGGCATCGCCAT-CATCGTC | 28 | GTCACCGGCTTCGTCTTCA | 29 | CAGGTCCACGGCAGTCT-GT | 30 |
| DCN | TCTTTTCAGCAACCCG-GTCCA | 31 | AAGGCTTCTTATTCGGGT-GTGA | 32 | TGGATGGCTGTATCTC-CCAGTA | 33 |
| DHCR7 | TGAGCGCCCACCCTCTC-GA | 34 | GGGCTCTGCTTCCCGATT | 35 | AGTCATAGGGCAAGCA-GAAAATTC | 36 |
| E2F8 | CAGGATACCTAATC-CCTCTCACGCAG | 37 | AAATGTCTCCGCAACCTT-GTTC | 38 | CTGCCCCCAGGGATGAG | 39 |
| EPHX2 | TGAAGCGGGAG-GACTTTTTGTAAA | 40 | CGATGAGAGTGTTTTATC-CATGCA | 41 | GCTGAGGCTGGGCTCT-TCT | 42 |
| ESR1 | ATGCCCTTTTGCCGATGCA | 43 | GCCAAATTGTGTTTGAT-GGATTAA | 44 | GACAAAACCGAGTCACAT-CAGTAATAG | 45 |
| GJA1 | TGCACAGCCTTTTGATTTC-CCCGAT | 46 | CGGGAAGCAC-CATCTCTAACTC | 47 | TTCATGTCCAG-CAGCTAGTTTTT | 48 |
| HSPA2 | CAAGTCAGCAAACACG-CAAAA | 49 | CATGCACGAACTAAT-CAAAAATGC | 50 | ACATTATTCGAGGTT-TCTCTTTAATGC | 51 |

EP 2 737 081 B1

(continued)

| gene | probe | Seq ID | forward primer | Seq ID | reverse primer | Seq ID |
|---|---|---|---|---|---|---|
| IL6ST | CAAGCTCCACCTTCCAAAGGACCT | 52 | CCCTGAATCCATAAAGGCATACC | 53 | CAGCTTCGTTTTCCCTACTTT-TT | 54 |
| INPP4B | TCCGAGCGCTGGATTGCATGAG | 55 | GCACCAGTTACACAAGGACTTCTTT. | 56 | TCTCTATGCGGCATCCTTCTC | 57 |
| MAPT | AGACTATTTGCACACTGCCGCCT | 58 | GTGGCTCAAAGGATAATATCAAACAC | 59 | ACCTTGCTCAGGTCAACTGGTT | 60 |
| MGP | CCTTCATATCCCCTCAGCAGAGATGG | 61 | CCTTCATTAACAGGAGAAATGCAA | 62 | ATTGAGCTCGTGGACAGGCTTA | 63 |
| NEK2 | TCCTGAACAAATGAATCGCATGTCCTACAA | 64 | ATTTGTTGGCACACCTTATTACATGT | 65 | AAGCAGCCCAATGACCAGATa | 66 |
| PCNA | AAATACTAAAATGCGCCGGCAATGA | 67 | GGGCGGTGAACCTCACCAGTA | 68 | CTTCGGCCCCTTAGTGTAATGATATC | 69 |
| PGR | TTGATAGAAACGCTGTGAGCTCGA | 70 | AGCTCATCAAGGCAATTGGTTT | 71 | ACAAGATCATGCAAGTTATCAAGAAGTT | 72 |
| PIP | TGCATGGTGGTTAAAACTTACCTCA | 73 | TGCTTGCAGTTCAAACAGAATTG | 74 | CACCTTGTAGAGGGATGCTGCTA | 75 |
| PLAT | CAGAAAGTGGCCATGCCACCCTG | 76 | TGGGAAGACATGAATGCACACTA | 77 | GGAGGGTTGGGCTTTAGdTGAA | 78 |
| PRSS16 | CACTGCCGGTCACCCACACCA | 79 | CTGAGGAGCACAGAACCTCAACT | 80 | CGAACTCGGTACACATGTCTGATACAA | 81 |
| PTGER3 | TCGGTCTGCTGGTCTCCGCTCC | 82 | CTGATTGAAGATCATTTTCAACATCA | 83 | GACGGCCATTCAGCTTATGG | 84 |
| PTPRT | TTGGCTTCTGGACACCCTCACA | 85 | GAGTTGTGGCCTCTACCATTGC | 86 | GAGCGGGAACCTTGGGATAG | 87 |
| RACGAP1 | ACTGAGAATCTCCACCCGGCGCA | 88 | TCGCCAACTGGATAAAATTGGA | 89 | GAATGTGCGGAATCTGTTTGAG | 90 |
| RBBP8 | ACCGATTCCGCTACATTCCACCCAAC | 91 | AGAAAATTGGCTTCCTGCTCAAG | 92 | AAAACCAACTTCCCAAAAATTCTCT | 93 |
| SCUBE2 | CTAGAGGGTTCCAGGTCCCATACGTGACATA | 94 | TGTGGATTCAGTTCAAGTCCAATG | 95 | CCATCTCGAACTATGTCTTCAATGAGT | 96 |
| SEC14L2 | TGGGAGGCATGCAACGCGTG | 97 | AGGTCTTACTAAGCAGTCCCATCTCT | 98 | CGACCGGCACCTGAACTC. | 99 |
| SQLE | TATGCGTCTCCCAAAAGAAGAACACCTCG | 100 | GCAAGCTTCCTTCCTCCTTCA | 101 | CCTTTAGCAGTTTTCTCCATAGTTTTATATC | 102 |

(continued)

| gene | probe | Seq ID | forward primer | Seq ID | reverse primer | Seq ID |
|---|---|---|---|---|---|---|
| TFAP2B | CAACACCACCACTAACAG-GCACACGTC | 103 | GGCATGGACAAGATGT-TCTTGA | 104 | CCTCCTTGTCGGCCAGTTT-TACT | 105 |
| TOP2A | CAGATCAGGACCAAGAT-GGTTCCCACAT | 106 | CATTGAAGACGCTTCGT-TATGG | 107 | CCAGTTGTGATGGA-TAAAATTAATCAG | 108 |
| TRIM29 | TGCTGTCTCACTACCG-GCCATTCTACG | 109 | TGGAAA-TCTGGCAAGCA-GACT | 110 | CAATCCCGTTGCCTTT-GTTG | 111 |
| UBE2C | TGAACACACATGCT-GCCGAGCTCTG | 112 | CTTCTAGGAGAACCCAA-CATTGATAGT | 113 | GTTTCTTGCAGGTACTTCT-TAAAAGCT | 114 |
| WNT5A | TATTCACATCCCCTCAGTT-GCAGTGAATTG | 115 | CTGTGGCTCTTAATTTATT-GCATAATG | 116 | TTAGTGCTTTTGCTT-TCAAGATCTT | 117 |
| STC2 | TCTCACCTTGACCCT-CAGCCAAG | 118 | ACATTTGACAAATTTCCCT-TAGGATT | 119 | CCAGGACGCAGCTTTAC-CAA | 120 |

A second alternative for unsupervised selection of possible gene replacement candidates is based on Affymetrix data only. This has the advantage that it can be done solely based on already published data (e.g. from www.ncbi.nlm.nih.gov/geo/). The following tables lists HG-U133a probe set replacement candidates for the probe sets used in algorithms T1 - T5. This is based on training data of these algorithms. The column header contains the gene name and the probe set ID in bold. Then, the 10 best-correlated probe sets are listed, where each table cell contains the probe set ID, the correlation coefficient in brackets and the gene name.

| UBE2C | BIRCS | DHCR7 | RACGAP1 | AURKA | PVALB | NMU | STC2 |
|---|---|---|---|---|---|---|---|
| **202954_at** | **202095_s_at** | **201791_s_at** | **222077_s_at** | **204092_s_at** | **205336_at** | **206023_at** | **203438_at** |
| 210052_s_at (0.82) TPX2 | 202954_at (0.82) UBE2C | 201790_s_at(0.66) DHCR7 | 218039_at (0.79) NUSAP1 | 208079_s_at (0.89) STK6 | 208663_at (-0.33) CAPN2 | 205347_s_at (0.45) TMSL8 | 203439_s_at (0.88) STC2 |
| 202095_s_at (0.82) BIRCS | 218039_at (0.81) NUSAP1 | 202218_s_at(0.48) FADS2 | 214710_s_at(0.78) CCNB1 | 202954_at (0.80) UBE2C | 219682_s_at (0.30) TBX3 | 203764_at (0.45) DLG7 | 212496_s_at ( 0.52) JMJD2B |
| 218009_s_at (0.82) PRC1 | 218009_s_at(0.79) PRC1 | 202580_x_at(0.47) FOXM1 | 203764_at (0.77) DLG7 | 210052_s_at ( 0.77) TPX2 | 218704_at (0.30) FU20315 | 203554_x_at (0.44) PTTG1 | 219440_at (0.52) RAI2 |
| 203554_x_at (0.82) PTTG1 | 202705_at (0.78) CCNB2 | 208944_at (-0.46) TGFBR2 | 204026_s_at(0.77) ZWINT | 202095_s_at ( 0.77) BIRC5 | | 204962_s_at (0.44) CENPA | 215867_x_at (0.51) CA12 |
| 208079_s_at (0.81) STK6 | 204962_s_at(0.78) CENPA | 202954_at (0.46) UBE2C | 218009_s_at(0.76) PRC1 | 203554_x_at (0.76) PTTG1 | | 204825_at ( 0.43) MELK | 214164_x_at ( 0.50) CA12 |
| 202705_at (0.81) CCNB2 | 203554_x_at(0.78) PTTG1 | 209541_at (-0.45) IGF1 | 204641_at (0.76) NEK2 | 218009_s_at (0.75) PRC1 | | 209714_s_at ( 0.41) CDKN3 | 204541_at ( 0.50) SEC14L2 |
| 218039_at (0.81) NUSAP1 | 208079_s_at ( 0.78) STK6 | 201059_at (0.45) CTTN | 204444_at ( 0.75) KIF11 | 201292_at ( 0.73) TOP2A | | 219918_s_at ( 0.41) ASPM | 203963_at (0.50) CA12 |
| 202870_s_at (0.80) CDC20 | 210052_s_at ( 0.77) TPX2 | 200795_at (-0.45) SPARCL1 | 202705_at (0.75) CCNB2 | 214710_s_at (0.73) CCNB1 | | 207828_s_at (0.41) CENPF | 212495_at (0.50) JMJD28 |
| 204092_s_at ( 0.80) STK6 | 202580_x_at ( 0.77) FOXM1 | 218009_s_at(0.45) PRC1 | 203362_s_at(0.75) MAD2L1 | 204962_s_at (0.73) CENPA | | 202705_at (0.41) CCNB2 | 208614_s_at (0.49) FLNB |
| 209408_at (0.80) KIF2C | 204092_s_at(0.77) STK6 | 218542_at ( 0.45) C10orf3 | 202954_at (0.75) UBE2C | 218039_at ( 0.73) NUSAP1 | | 219787_s_at ( 0.40) ECT2 | 213933_at ( 0.49) PTGER3 |

EP 2 737 081 B1

| 4ZGP1 | RBBP8 | IL6ST | MGP | PTGER3 CXCL12 ABAT | | | CDH1 |
|---|---|---|---|---|---|---|---|
| 209309_at | 203344_s_at | 212196_at | 202291_s_at | 213933_at | 209687_at | 209460_at | 201131 s_at |
| 217014_s_at ( 0.92) AZGP1 | **36499_at.** ( 0.49) **CELSR2** | 212195_at ( 0.85) IL6ST | 201288_at (0.46) ARHGDIB | 210375_at ( 0.74) PTGER3 | 204955_at (0.81) SRPX | 209459_s_at (0.92) ABAT | 201130_s_at (0.57) CDH1 |
| 206509_at ( 0.52) PIP | 204029_at ( 0.45) CELSR2 | 204864_s_at ( 0.75) IL6ST | 219768_at (0.42) VTCN1 | 210831_s_at (0.74) PTGER3 | 209335_at (0.81) DCN | 206527_at (0.63) ABAT | 221597_s_at (0.40) HSPC171 |
| 204541_at ( 0.46) SEC14L2 | 208305_at ( 0.45) PGR | 211000_s_at ( 0.68) I16ST | 202849_x_at (-0.41) GRK6 | 210374_x_at (0.73) PTGER3 | 211896_s_at (0.81) DCN | 213392_at ( 0.54) MGC35048 | 203350_at (0.38) AP1G1 |
| 200670_at (0.45) XBP1 | 205380_at ( 0.43) PDZK1 | 214077_x_at (0.61) MEIS4 | 205382_s_at (0.40) DF | 210832_x_at (0.73) PTGER3 | 201893_x_at (0.81) DCN | 221666_s_at ( 0.49) PYCARD | 209163_at (0.36) CYB561 |
| 209368_at (0.45) EPHX2 | 203303_at ( 0.41) TCTE1L | 204863_s_at ( 0.58) IL6ST | 200099_s_at (0.39) RPS3A | 210834_s_at ( 0.55) PTGER3 | 203666_at ( 0.80) CXCL12 | 218016_s_at ( 0.48) POLR3E | 210239_at (0.35) IRX5 |
| 218627_at (-0.43) FU11259 | 205280_at ( 0.38) GLRB | 202089_s_at (0.57) SLC39A6 | 221591_s_art (-0.37) FAM64A | 210833_at ( 0.55) PTGER3 | 211813_x_at ( 0.80) DCN | 214440_at (0.46) NAT1 | 200942_s_at ( 0.34) HSBP1 |
| 202286_s_at (0.43) TACSTD2 | 205279_s_at ( 0.38) GLRB | 210735_s_at ( 0.56) CA12 | 214629_x_at (0.37) RTN4 | 203438_at ( 0.49) STC2 | 208747_s_at (0.79) C1S | 204981_at ( 0.45) SLC22A18 | 209157_at (0.34) DNAJA2 |
| 213832_at ( 0.42) | 203685_at ( 0.38) BCL2 | 200648_s_at ( 0.52) GLUL | 200748_s_at (0.37) FTH1 | 203439_s_at (0.46) STC2 | 203131_at ( 0.78) PDGFRA | 212195_at (0.45) IL6ST | 210715_s_at (0.33) SPINT2 |
| 204288_s_at ( 0.41) SORBS2 | 203304_at (-0.39) BAMBI | 214552_s_at (0.52) RABEP1 | 209408_at (-0.37) KIF2C | 212195_at ( 0.41) IL6ST | 202994_s_at (0.78) FBLN1 | 204497_at (0.45) ADCY9 | 203219_s_at ( 0.33) APRT |
| 202376_at ( 0.41) SERPINA3 | 205862_at (0.36) GREB1 | 219197_s_at (0.51) SCUBE2 | 218726_at (-0.36) DKFZp762E1312 | 217764_s_at ( 0.40) RAB31 | 208944_at ( 0.78) TGFBR2 | 215867_x_at ( 0.45) CA12 | 218074_at ( 0.33) FAM96B |

EP 2 737 081 B1

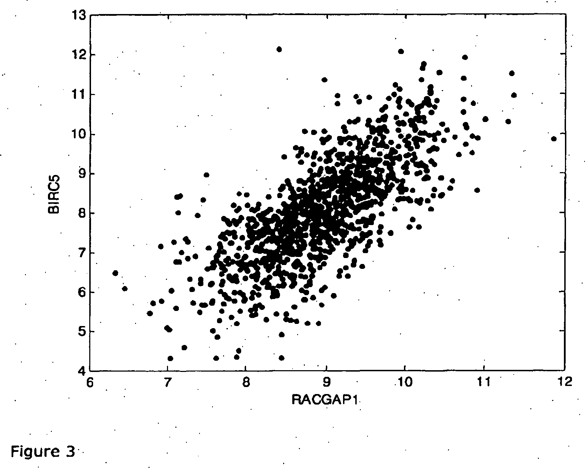

After selection of a gene or a probe set one has to define a mathematical mapping between the expression values of the gene to replace and those of the new gene. There are several alternatives which are discussed here based on the example "replace delta-Ct values of BIRC5 by RACGAP1". In the training data the joint distribution of expressions looks like Figure 3.

The Pearson correlation coefficient is 0.73.

One approach is to create a mapping function from RACGAP1 to BIRC5 by regression. Linear regression is the first choice and yields in this example

$$BIRC5 = 1.22 * RACGAP1 - 2.85.$$

Using this equation one can easily replace the BIRC5 variable in e.g. algorithm T5 by the right hand side. In other examples robust regression, polynomial regression or univariate nonlinear pre-transformations may be adequate.

The regression method assumes measurement noise on BIRC5, but no noise on RACGAP1. Therefore the mapping is not symmetric with respect to ex-changeability of the two variables. A symmetric mapping approach would be based on two univariate z-transformations.

$$z = (BIRC5 - mean(BIRC5)) / std(BIRC5) \text{ and}$$

$$z = (RACGAP1 - mean(RACGAP1)) / std(RACGAP1)$$

$$z = (BIRC5 - 8.09) / 1.29 = (RACGAP1 - 8.95) / 0.77$$

$$BIRC5 = 1.67 * RACGAP1 + -6.89$$

Again, in other examples, other transformations may be adequate: normalization by median and/or mad, nonlinear mappings, or others.

$$z = (BIRC5 - mean(BIRC5)) / std(BIRC5) \text{ and}$$

$$z = (RACGAP1 - mean(RACGAP1)) / std(RACGAP1)$$

$$z = (BIRC5 - 8.09) / 1.29 = (RACGAP1 - 8.95) / 0.77$$

$$BIRC5 = 1.67 * RACGAP1 + -6.89$$

Again, in other examples, other transformations may be adequate: normalization by median and/or mad, nonlinear mappings, or others.

**Claims**

1. A method for predicting a response to and/or benefit of chemotherapy with taxane or anthracycline, including neoadjuvant chemotherapy, in a patient suffering from or at risk of developing recurrent breast cancer, said method comprising the steps of:

    (a) determining in a breast tumor sample from said patient the RNA expression levels of the following 8 genes: UBE2C, RACGAP1, DHCR7, STC2, AZGP1, RBBP8, IL6ST, and MGP, indicative of a response to chemotherapy for a tumor, or

(b) determining in a breast tumor sample from said patient the RNA expression levels of the following 8 genes: UBE2C, BIRC5, DHCR7, STC2, AZGP1, RBBP8, IL6ST, and MGP; indicative of a response to chemotherapy for a tumor

(c) mathematically combining expression level values for the genes of the said set which values were determined in the tumor sample to yield a combined score, wherein said combined score is predicting said response and/or benefit of chemotherapy.

2. The method of any one of the foregoing claims, wherein said expression level is determined as a non-protein such as a gene expression level.

3. The method of any one of the foregoing claims, wherein said expression level is determined by at least one of

a PCR based method,
a micorarray based method, or
a hybridization based method, a sequencing and/or next generation sequencing approach.

4. The method of any one of the foregoing claims, wherein said determination of expression levels is in a formalin-fixed paraffin-embedded tumor sample or in a fresh-frozen tumor sample.

5. The method of any one of the foregoing claims, wherein the expression level of said at least one marker gene is determined as a pattern of expression relative to at least one reference gene or to a computed average expression value.

6. The method of any one of the foregoing claims, wherein said step of mathematically combining comprises a step of applying an algorithm to values representative of an expression level of a given gene, in particular wherein said algorithm is a linear combination of said. values representative of an expression level of a given gene, or wherein a value for a representative of an expression level of a given gene is multiplied with a coefficient.

7. The method of any one of the foregoing claims, wherein one, two or more thresholds are determined for said combined score and discriminated into high and low risk, high, intermediate and low risk, or more risk groups by applying the threshold on the combined score.

8. The method of any one of the foregoing claims, wherein a high combined score is indicative of benefit from a more aggressive therapy, e.g. cytotoxic chemotherapy.

9. The method of any one of the foregoing claims, wherein information regarding nodal status of the patient is processed in the step of mathematically combining expression level values for the genes to yield a combined score.

10. The method of any one of the foregoing claims, wherein said information regarding tumor size of the patient is processed in the step of mathematically combining expression level values for the genes to yield a combined score.

11. The method of any one of the foregoing claims, wherein said information regarding nodal status and tumor size of the patient is processed in the step of mathematically combining expression level values for the genes to yield a combined score.

12. Use of a kit for performing the method of at least one of the claims 1 to 11, said kit comprising a set of oligonucleotides capable of specifically binding sequences or to sequences of fragments of the genes in a combination of genes, wherein

(i) said combination comprises at least the 8 genes UBE2C, BIRC5, DHCR7, STC2, AZGP1, RBBP8, IL6ST, and MGP; or
(ii) said combination comprises at least the 8 genes UBE2C, RACGAP1, DHCR7, STC2, AZGP1, RBBP8, IL6ST, and MGP.

**Patentansprüche**

1. Verfahren zur Vorhersage eines Ansprechens auf und/oder Nutzens einer Chemotherapie mit Taxan oder Anthracyclin einschließlich einer Neoadjuvans-Chemotherapie bei einem Patienten, der an rezidivierendem Brustkrebs leidet oder bei dem das Risiko besteht, dass er einen solchen entwickelt, wobei das Verfahren die folgenden Schritte umfasst:

(a) Bestimmen der RNA-Expressionsniveaus der folgenden 8 Gene in einer Brusttumorprobe von dem Patienten: UBE2C, RACGAP1, DHCR7, STC2, AZGP1, RBBP8, IL6ST und MGP, die ein Ansprechen auf Chemotherapie für einen Tumor anzeigen; oder

(b) Bestimmen der RNA-Expressionsniveaus der folgenden 8 Gene in einer Brusttumorprobe von dem Patienten: UBE2C, BIRC5, DHCR7, STC2, AZGP1, RBBP8, IL6ST und MGP, die ein Ansprechen auf Chemotherapie für einen Tumor anzeigen;

(c) mathematisches Kombinieren der Expressionsniveauwerte für die Gene der genannten Menge, wobei die Werte in der Tumorprobe bestimmt wurden, unter Erhalt eines kombinierten Score, wobei der kombinierte Score das Ansprechen auf und/oder den Nutzen einer Chemotherapie vorhersagt.

2. Verfahren gemäß einem der vorstehenden Ansprüche, wobei das Expressionsniveau als Nichtprotein, wie auf dem Genexpressionsniveau, bestimmt wird.

3. Verfahren gemäß einem der vorstehenden Ansprüche, wobei das Expressionsniveau durch wenigstens eines der folgenden Verfahren bestimmt wird:

ein Verfahren auf PCR-Basis;
ein Verfahren auf Mikroarray-Basis; oder
ein Verfahren auf Hybridisierungsbasis, einen Sequenzierungs- und/oder Nächste-Generation-Sequenzierungsansatz.

4. Verfahren gemäß einem der vorstehenden Ansprüche, wobei die Bestimmung der Expressionsniveaus in einer mit Formalin fixierten und in Paraffin eingebetteten Tumorprobe oder in einer frisch eingefrorenen Tumorprobe erfolgt.

5. Verfahren gemäß einem der vorstehenden Ansprüche, wobei das Expressionsniveau des wenigstens einen Marker-Gens als Expressionsmuster relativ zu wenigstens einem Referenz-Gen oder zu einem berechneten mittleren Expressionswert bestimmt wird.

6. Verfahren gemäß einem der vorstehenden Ansprüche, wobei der Schritt des mathematischen Kombinierens einen Schritt des Anwendens eines Algorithmus auf Werte, die repräsentativ für ein Expressionsniveau eines gegebenen Gens sind, umfasst, wobei der Algorithmus insbesondere eine Linearkombination der Werte ist, die repräsentativ für ein Expressionsniveau eines gegebenen Gens sind, oder wobei ein Wert, der repräsentativ für ein Expressionsniveau eines gegebenen Gens ist, mit einem Koeffizienten multipliziert wird.

7. Verfahren gemäß einem der vorstehenden Ansprüche, wobei eine, zwei oder mehr Schwellen für den kombinierten Score bestimmt werden, die zwischen hohen und geringem Risiko, hohem, mittlerem und geringem Risiko oder mehr Risikogruppen diskriminieren, indem man die Schwelle auf den kombinierten Score anwendet.

8. Verfahren gemäß einem der vorstehenden Ansprüche, wobei ein hoher kombinierter Score den Nutzen einer aggressiveren Therapie, zum Beispiel einer cytotoxischen Chemotherapie, anzeigt.

9. Verfahren gemäß einem der vorstehenden Ansprüche, wobei in dem Schritt des mathematischen Kombinierens der Expressionsniveauwerte für die Gene Informationen bezüglich des Nodalstatus des Patienten verarbeitet werden, wobei man einen kombinierten Score erhält.

10. Verfahren gemäß einem der vorstehenden Ansprüche, wobei in dem Schritt des mathematischen Kombinierens der Expressionsniveauwerte für die Gene Informationen bezüglich der Tumorgröße des Patienten verarbeitet werden, wobei man einen kombinierten Score erhält.

11. Verfahren gemäß einem der vorstehenden Ansprüche, wobei in dem Schritt des mathematischen Kombinierens der Expressionsniveauwerte für die Gene Informationen bezüglich des Nodalstatus und der Tumorgröße des Patienten verarbeitet werden, wobei man einen kombinierten Score erhält.

12. Verwendung eines Kits zur Durchführung des Verfahrens gemäß wenigstens einem der Ansprüche 1 bis 11, wobei der Kit eine Menge von Oligonucleotiden umfasst, die spezifisch Sequenzen oder an Sequenzen von Fragmenten der Gene in einer Kombination von Genen binden können, wobei

(i) die Kombination wenigstens die 8 Gene UBE2C, BIRC5, DHCR7, STC2, AZGP1, RBBP8, IL6ST und MGP umfasst; oder
(ii) die Kombination wenigstens die 8 Gene UBE2C, RACGAP1, DHCR7, STC2, AZGP1, RBBP8, IL6ST und MGP umfasst.

**Revendications**

1. Méthode de prédiction d'une réponse à et/ou d'un bénéfice d'une chimiothérapie avec le taxane ou l'anthracycline, incluant une chimiothérapie néoadjuvante, chez un patient souffrant de ou présentant. un risque de développer un cancer du sein récurrent, ladite méthode comprenant les étapes consistant à :

(a) déterminer dans un échantillon de tumeur du sein issu dudit patient des niveaux d'expression d'ARN des 8 gènes suivants : UBE2C, RACGAP1, DHCR7, STC2, AZGP1, RBBP8, IL6ST, et MGP, indiquant une réponse à une chimiothérapie contre une tumeur, ou
(b) déterminer dans un échantillon de tumeur du sein issu dudit patient des niveaux d'expression d'ARN des 8 gènes suivants : UBE2C, BIRC5, DHCR7, STC2, AZGP1, RBBP8, IL6ST, et MGP, indiquant réponse à une chimiothérapie contre une tumeur
(c) combiner mathématiquement les valeurs de niveau d'expression pour les gènes dudit ensemble, lesquelles valeurs ont été déterminées dans l'échantillon de tumeur pour produire un score combiné, ledit score combiné prédisant ladite réponse et/ou ledit bénéfice de la chimiothérapie.

2. Méthode selon l'une quelconque des revendications précédentes, dans laquelle ledit niveau d'expression est déterminé comme une non-protéine telle qu'un niveau d'expression de gène.

3. Méthode selon l'une quelconque des revendications précédentes, dans laquelle ledit niveau d'expression est déterminé par au moins l'une parmi
une méthode basée sur la PCR,
une méthode basée sur les microréseaux, ou
une méthode basée sur l'hybridation, une approche de séquençage et/ou de séquençage de nouvelle génération.

4. Méthode selon l'une quelconque des revendications précédentes, dans laquelle ladite détermination des niveaux d'expression se fait dans un échantillon de tumeur inclus dans la paraffine et fixé à la formaline ou dans un échantillon de tumeur congelé frais.

5. Méthode selon l'une quelconque des revendications précédentes, dans laquelle le niveau d'expression dudit au moins un gène marqueur est déterminé comme un motif d'expression relatif à au moins un gène de référence ou à une valeur d'expression moyenne calculée.

6. Méthode selon l'une quelconque des revendications précédentes, dans laquelle ladite étape de combinaison mathématique comprend une étape consistant à appliquer un algorithme à des valeurs représentatives d'un niveau d'expression d'un gène donné, en particulier dans laquelle ledit algorithme est une combinaison linéaire desdites valeurs représentatives d'un niveau d'expression d'un gène donné, ou dans laquelle, une valeur pour un niveau d'expression représentatif d'un gène donné est multipliée par un coefficient.

7. Méthode selon l'une quelconque des revendications précédentes, dans laquelle un, deux ou plusieurs seuils sont déterminés pour ledit score combiné et discriminés en groupes à risque élevé et faible, à risque élevé, intermédiaire et faible, ou à plus de risque en appliquant le seuil sur le score combiné.

8. Méthode selon l'une quelconque des revendications précédentes, dans laquelle un score combiné élevé est indicatif d'un bénéfice apporté par une thérapie plus agressive, par exemple une chimiothérapie cytotoxique.

9. Méthode selon l'une quelconque des revendications précédentes, dans laquelle des informations à propos d'un statut nodal du patient sont traitées dans l'étape de combinaison mathématique de valeurs de niveau d'expression pour les gènes pour produire un score combiné.

**10.** Méthode selon l'une quelconque des revendications précédentes, dans laquelle lesdites informations à propos de la taille de la tumeur du patient sont traitées dans l'étape de combinaison mathématique de valeurs de niveau d'expression pour les gènes pour produire un score combiné.

**11.** Méthode selon l'une quelconque des revendications précédentes, dans laquelle lesdites informations à propos du statut nodal et de la taille de la tumeur du patient sont traitées dans l'étape de combinaison mathématique de valeurs de niveau d'expression pour les gènes pour produire un score combiné.

**12.** Utilisation d'un kit permettant de réaliser la méthode d'au moins l'une des revendications 1 à 11, ledit kit comprenant un jeu d'oligonucléotides capable de se lier spécifiquement à des séquences ou à des séquences de fragments des gènes dans une combinaison de gènes, dans laquelle

(i) ladite combinaison comprend au moins les 8 gènes UBE2C, BIRC5, DHCR7, STC2, AZGP1, RBBP8, IL6ST, et MGP ; ou
(ii) ladite combinaison comprend au moins les 8 gènes UBE2C, RACGAP1, DHCR7, STC2, AZGP1, RBBP8, IL6ST, et MGP.

Figure 1

Figure 2

Figure 3

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2010076322 A1
- WO 2009158143 A1
- WO 2006119593 A

- WO 2008006517 A2
- WO 2009114836 A1
- WO 2011120984 A1


**Non-patent literature cited in the description**

- **JEMAL et al.** *CA Cancer J Clin.,* 2011
- **MISSET et al.** *J Clin Oncol.,* 1996
- **HENDERSON et al.** *J Clin Oncol.,* 2003
- **HESS et al.** *J Clin Oncol.,* 2006
- **TABCHY et al.** *Clin Can Res,* 2010
- **SOTIRIOU et al.** *JNCI,* 2006

- **LIEDTKE.** *J Clin Oncol,* 2009
- **MAIA CHANRION et al.** *report in Clin Cancer Res,* 15 March 2008, vol. 14 (6), 1744-1752
- **KAREN J TAYLOR et al.** *report in Breast Cancer Research,* 2010, vol. 12, R39